# EUROPEAN PATENT SPECIFICATION

(11) **EP 2 898 817 B1**
(45) Date of publication and mention of the grant of the patent: **01.11.2017**
(21) Application number: 13839074.5
(22) Date of filing: 11.09.2013
(51) Int. Cl.: A61B 1/00

(54) **INSERTION/EXTRACTION-AIDING MEANS**
HILFSVORRICHTUNG FÜR EINSATZ/EXTRAKTION
MOYEN D'AIDE À L'INSERTION/EXTRACTION

(30) Priority: 19.09.2012 JP 2012206171; 20.09.2012 JP 2012207568
(43) Date of publication of application: 29.07.2015
(73) Proprietor: Olympus Corporation, Tokyo 192-8507 (JP)
(72) Inventor: NISHIIE, Takehiro, Hachioji-shi Tokyo 192-8512 (JP); NAITO, Kimihiko, Hachioji-shi Tokyo 192-8512 (JP); MIYOSHI, Hiroaki, Hachioji-shi Tokyo 192-8512 (JP)
(74) Representative: Gunzelmann, Rainer
(86) International application number: PCT/JP2013/074598
(87) International publication number: WO 2014/045980

(56) References cited:
- WO-A1-2007/080899
- WO-A1-2011/140118
- JP-A- 2002 514 111
- JP-A- 2010 523 208

## Description

### Technical Field

The present invention relates to an auxiliary insertion and removal device that assists insertion and removal of an inserting section into and from a lumen.

### Background Art

For example, Patent Literature 1 discloses a catheter. This catheter has a corrugated tube into which an endoscope is insertable, and a spiral thread arranged in a distal end portion of the corrugated tube. Furthermore, this catheter also has a handle arranged away from the spiral thread and arranged in a proximal end portion of the corrugated tube to rotate the spiral thread through the corrugated tube, and a fixing portion arranged in the proximal end portion of the corrugated tube to the handle to fix the endoscope.

The corrugated tube has a cylindrical shape. The spiral thread is arranged on an outer peripheral surface of the corrugated tube and spirally arranged around a longitudinal axis of the corrugated tube. When the handle rotates, the corrugated tube rotates. Furthermore, when the corrugated tube rotates, the spiral thread rotates. The rotating spiral thread engages with an inner wall of a lumen, so that the corrugated tube receives a propulsive force. As a result, for example, the corrugated tube is assisted to move forward by this propulsive force.

### Citation List

### Patent Literature

Patent Literature 1: Jpn. PCT National Publication No. 2011-520563

### Summary of Invention

### Technical Problem

For example, when a corrugated tube is inserted from a stomach toward a duodenum, the corrugated tube passes a pylorus. The duodenum and pylorus are thinner as compared with the stomach, and the corrugated tube is not easily inserted into the pylorus when a position of the corrugated tube is not confirmed. Furthermore, when the position of the corrugated tube is not confirmed, the corrugated tube disadvantageously abuts on a stomach wall before the corrugated tube is inserted into the pylorus, hence it is feared that the stomach wall will be damaged.

Furthermore, when the corrugated tube passes the pylorus, a spiral thread and the corrugated tube receive resistance from the pylorus. Therefore, when the position of the corrugated tube is not confirmed, it is wrongly recognized that the corrugated tube abuts on the stomach wall, though the corrugated tube passes the pylorus and is inserted into the duodenum, hence it is feared that the corrugated tube will be wrongly removed from the pylorus.

In consequence, it is demanded to easily confirm, for example, at which position in a body the corrugated tube is disposed.

Furthermore, when the corrugated tube passes the pylorus, rotation of the corrugated tube is stopped by the above resistance, or a speed of the rotation is lowered by the resistance. Thus it is feared that a rotational situation will change due to the resistance. At this time, an endoscope is inserted into the corrugated tube, and the spiral thread and the corrugated tube are not imaged in an image acquired by the endoscope. Therefore, it is feared that the change of the rotational situation cannot easily be confirmed from the image acquired by the endoscope.

It is to be noted that a catheter is usually used under X-ray observation. At this time, the catheter is not clearly imaged by X-rays. Therefore, under X-ray observation, the rotational situations of the spiral thread and the corrugated tube are not clearly displayed in a monitor or the like, hence it is feared that the rotational situations cannot easily be confirmed.

As described above, under X-ray observation, it is feared that the positions of the spiral thread and the corrugated tube and the rotational situations of the spiral thread and the corrugated tube cannot easily be confirmed, and it is demanded that the confirmation is easily performed.

It is to be noted that when the corrugated tube is removed from the stomach toward an esophagus, the corrugated tube passes the pylorus. Also at this time, there is the fear that the positions of the spiral thread and the corrugated tube and the rotational situations of the spiral thread and the corrugated tube cannot easily be confirmed, and it is demanded for the confirmation to be easily performed.

In consequence, it is an object of the present invention to provide an auxiliary insertion and removal device that can easily confirm positions and rotational situations under X-ray observation.

### Solution to Problem

The present invention is defined by independent claim 1. Specified embodiments are defined by the dependent claims.

An aspect of an auxiliary insertion and removal device that is inserted into or removed from a lumen in a state where an inserting section of an endoscope having a longitudinal axis is inserted thereinto and that assists in the insertion and the removal of the inserting section into and from the lumen, the auxiliary insertion and removal device including a tubular main body section that allows the inserting section to be inserted thereinto and rotates around the longitudinal axis; a fin section that is disposed on an outer peripheral surface of the main body section and spirally arranged around the longitudinal axis; and an X-ray cutoff section that is arranged in at least a part of the auxiliary insertion and removal device in a longitudinal axis direction of the auxiliary insertion and removal device, and arranged so that a clear region to be clearly imaged by X-rays and an unclear region to be unclearly imaged by X-rays are distinguished under X-ray observation in a peripheral direction of the auxiliary insertion and removal device, to cut off X-rays.

### Advantageous Effects of Invention

According to the present invention, it is possible to provide the auxiliary insertion and removal device that can easily confirm positions and rotational situations under X-ray observation.

### Brief Description of Drawings

FIG. 1 is a schematic block diagram of an endoscope system according to a first embodiment of the present invention;
FIG. 2 is a side elevation of an operating section seen from a bending operating section side;
FIG. 3A is a perspective view showing a coupling configuration of a proximal end portion of a bending section and a distal end portion of a flexible tube section;
FIG. 3B is a cross-sectional view showing the coupling configuration of the proximal end portion of the bending section and the distal end portion of the flexible tube section;
FIG. 3C is a cross-sectional view taken along a line 3C-3C depicted in FIG. 3B;
FIG. 4A is a perspective view of a spiral rotary member;
FIG. 4B is a schematic view to explain an arrangement position of an X-ray cutoff section in a periaxial direction of a longitudinal axis of the spiral rotary member;
FIG. 5A is a view of a state where the spiral rotary member moves forward from a stomach to a pylorus;
FIG. 5B is a view showing a state where the spiral rotary member depicted in FIG. 5A is imaged, and explaining a position and a rotational situation of a distal end portion side of the spiral rotary member;
FIG. 6A is a view of a state where the spiral rotary member is inserted into a pylorus;
FIG. 6B is a view showing a state where the spiral rotary member depicted in FIG. 6A is imaged, and explaining a position and a rotational situation of a proximal end portion side of the spiral rotary member;
FIG. 7 is a perspective view of an overtube;
FIG. 8 is a schematic view showing an endoscope device according to a second embodiment of the present invention;
FIG. 9 is a perspective view schematically showing an auxiliary insertion device according to the second embodiment;
FIG. 10 is a cross-sectional view schematically showing the auxiliary insertion device according to the second embodiment which is attached to an inserting section;
FIG. 11 is a cross-sectional view schematically showing a configuration in a non-regulated state of a tubular main body and a fixing tubular section of the auxiliary insertion device according to the second embodiment;
FIG. 12 is a cross-sectional view schematically showing a configuration in a regulated state of the tubular main body and the fixing tubular section of the auxiliary insertion device according to the second embodiment;
FIG. 13 is a schematic view to explain a state where a rotary tubular section of an auxiliary insertion device according to the first embodiment rotates in a periaxial direction of a rotation axis in a lumen;
FIG. 14 is a cross-sectional view schematically showing a configuration in a non-regulated state of a tubular main body and a fixing tubular section of an auxiliary insertion device according to a first modification;
FIG. 15 is a cross-sectional view schematically showing a configuration in a regulated state of the tubular main body and the fixing tubular section of the auxiliary insertion device according to the first modification;
FIG. 16 is a perspective view schematically showing that an auxiliary insertion device according to a third embodiment is attached to an inserting section;
FIG. 17 is a cross-sectional view schematically showing that the auxiliary insertion device according to the third embodiment is attached to the inserting section;
FIG. 18A is a cross-sectional view schematically showing a configuration of a tubular main body and a fixing tubular section of the auxiliary insertion device according to the third embodiment;
FIG. 18B is a schematic view showing a state where the inserting section and the auxiliary insertion device according to the third embodiment are inserted from a mouth into a lumen;
FIG. 19 is a cross-sectional view schematically showing a configuration of a tubular main body and a rotary tubular section of an auxiliary insertion device according to a second modification;
FIG. 20 is a schematic view to explain a configuration of a tubular main body of an auxiliary insertion device according to a third modification; and
FIG. 21 is a cross-sectional view schematically showing a configuration of a drive member and a tubular main body of an auxiliary insertion device according to a fourth modification.

### Brief Description of Embodiments

Hereinafter, embodiments of the present invention will be described in detail with reference to the drawings.

### [First Embodiment]

### [Configuration]

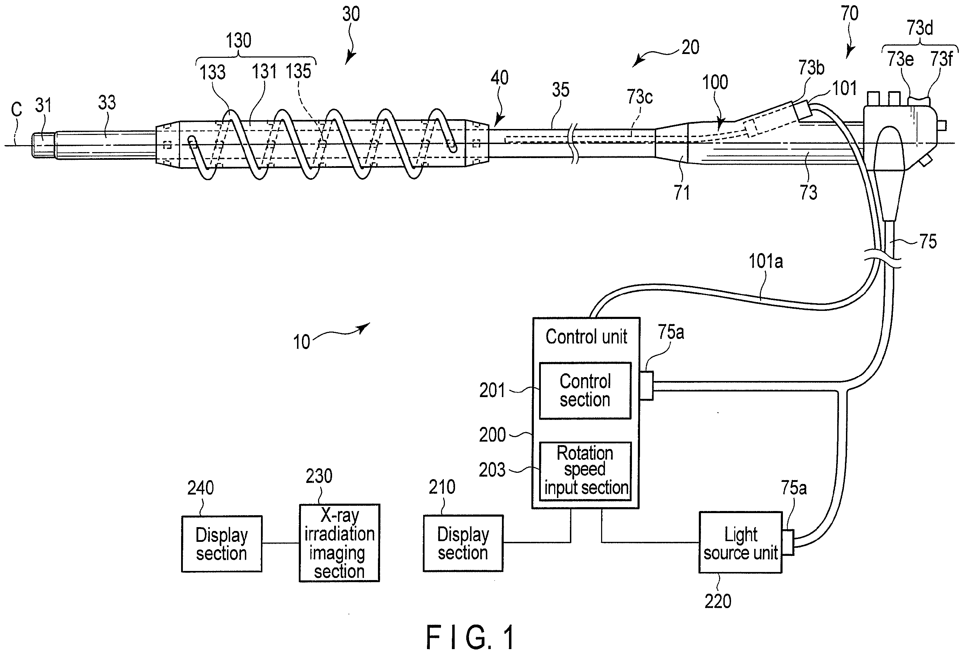

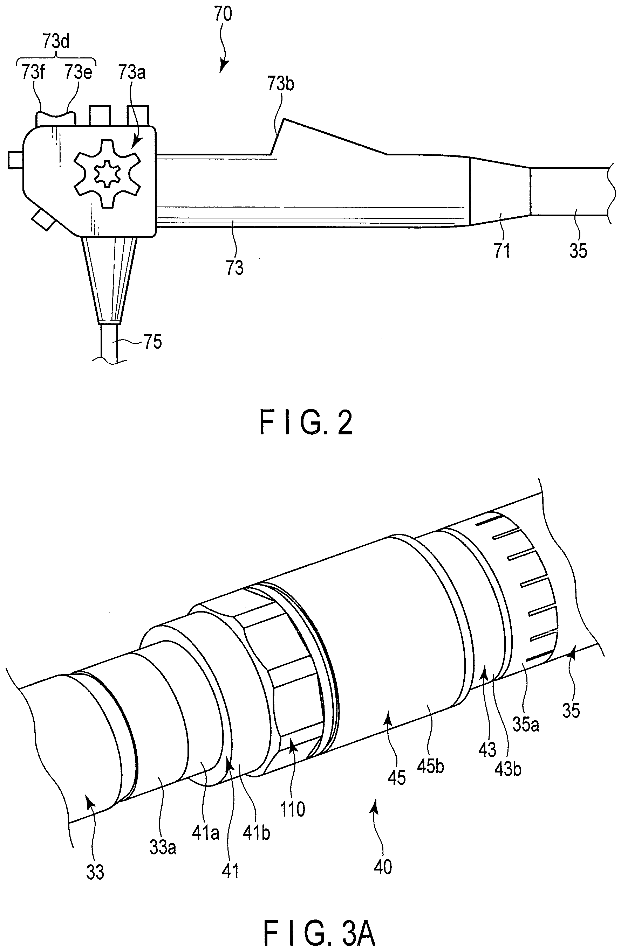

In reference to FIG. 1, FIG. 2, FIG. 3A, FIG. 3B, FIG. 3C, FIG. 4A, FIG. 4B, FIG. 5A, FIG. 5B, FIG. 6A, and FIG. 6B, a first embodiment will be described. It is to be noted that some members are omitted in some of the drawings to clarify the drawings; for example, where a drive member 101 and a cable 101a are omitted in FIG. 2. Furthermore, in the following description, a longitudinal axis means a longitudinal axis C of an inserting section 30. A longitudinal axis direction means, e.g., a longitudinal axis direction of the inserting section 30. A radial direction means a radial direction of the inserting section 30.

### [Endoscope System 10]

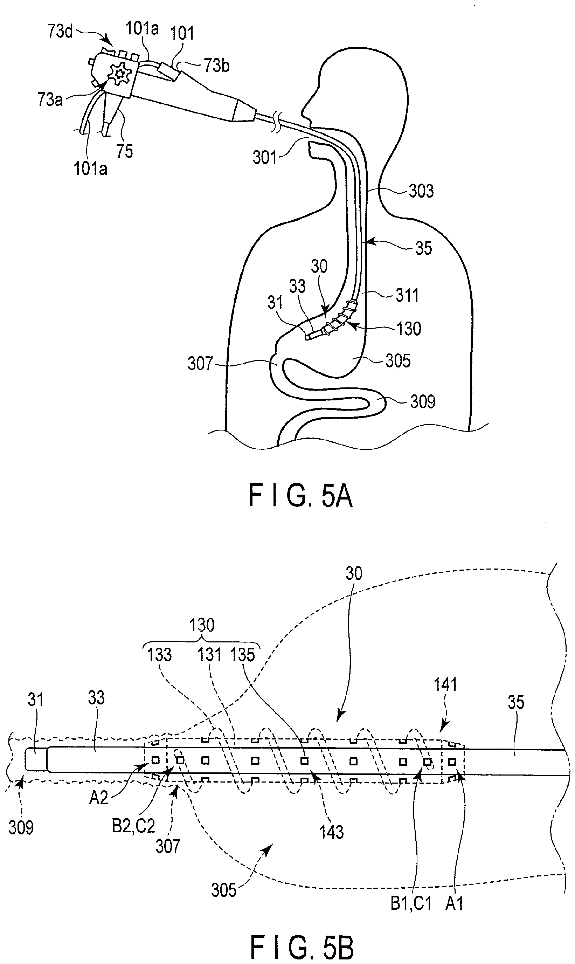

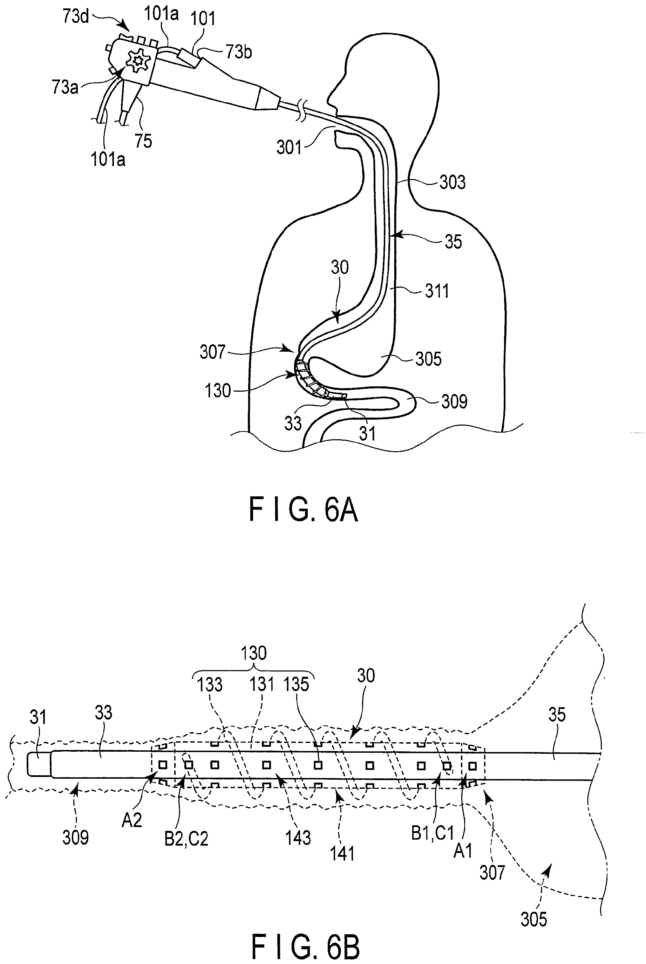

As shown in FIG. 1, an endoscope system 10 has an endoscope 20 having the inserting section 30 that is inserted into or removed from, e.g., a lumen of a subject, and a control unit 200 that controls a propulsive force to assist the insertion and the removal when the inserting section 30 is inserted into or removed from the lumen. As shown in FIG. 5A, FIG. 5B, FIG. 6A, and FIG. 6B, the lumen means, e.g., a pylorus 307, a duodenum 309, a cardiac orifice 311, or the like.

Further, as shown in FIG. 1, the endoscope system 10 also has a display section 210 that displays an image acquired by the endoscope 20 and a light source unit 220 arranged to emit light from a distal end portion of the inserting section 30 to an observation target. The image acquired by the endoscope 20 shows the observation target in the lumen. The observation target is, e.g., an affected part or a lesioned part in the lumen.

Further, as shown in FIG. 1, the endoscope system 10 is used together with an X-ray irradiation imaging section 230 arranged in, e.g., an endoscope inspection room to emit, e.g., X-rays, thereby forming an image, and a display section 240 arranged together with the X-ray irradiation imaging section 230 in, e.g., the endoscope inspection room to display the image formed by the X-ray irradiation imaging section 230.

### [Configuration 1 of Endoscope 20]

As shown in FIG. 1, the endoscope 20 has the elongated inserting section 30 that is inserted into or removed from the lumen and has the longitudinal axis C, and an operating section 70 that is coupled with a proximal end portion of the inserting section 30 and operates the endoscope 20. The endoscope 20 can be cleaned and sterilized.

### [Inserting Section 30]

As shown in FIG. 1, the inserting section 30 has a distal end hard section 31, a bending section 33, and a flexible tube section 35 from a distal end portion side of the inserting section 30 toward a proximal end portion side of the inserting section 30. A proximal end portion of the distal end hard section 31 is coupled with a distal end portion of the bending section 33, and a proximal end portion of the bending section 33 is coupled with a distal end portion of the flexible tube section 35.

The distal end hard section 31 is the distal end portion of the inserting section 30, and it is hard and does not bend. The distal end hard section 31 has a non-illustrated imaging unit that images the observation target and a non-illustrated emitting section that emits light toward the observation target. That emitting section is optically connected to the light source unit 220 and emits the light guided from the light source unit 220 toward the observation target.

The bending section 33 bends in desired directions, e.g., upper and lower directions by an operation of a later-described bending operating section 73a shown in FIG. 2. When the bending section 33 bends, a position and a direction of the distal end hard section 31 vary. As a result, the non-illustrated observation target is illuminated with the light, and the non-illustrated observation target is captured in an observation viewing field.

The flexible tube section 35 has desirable flexibility. Therefore, the flexible tube section 35 bends due to an external force. The flexible tube section 35 functions as a tubular member extending from a later-described main body section 71 in the operating section 70. The flexible tube section 35 has, e.g., a spiral tube made of a metal, a reticular tube that is arranged on an outer side of this spiral tube and covers the spiral tube, and an outer coat that is made of a resin and arranged on an outer side of this reticular tube and covers the reticular tube.

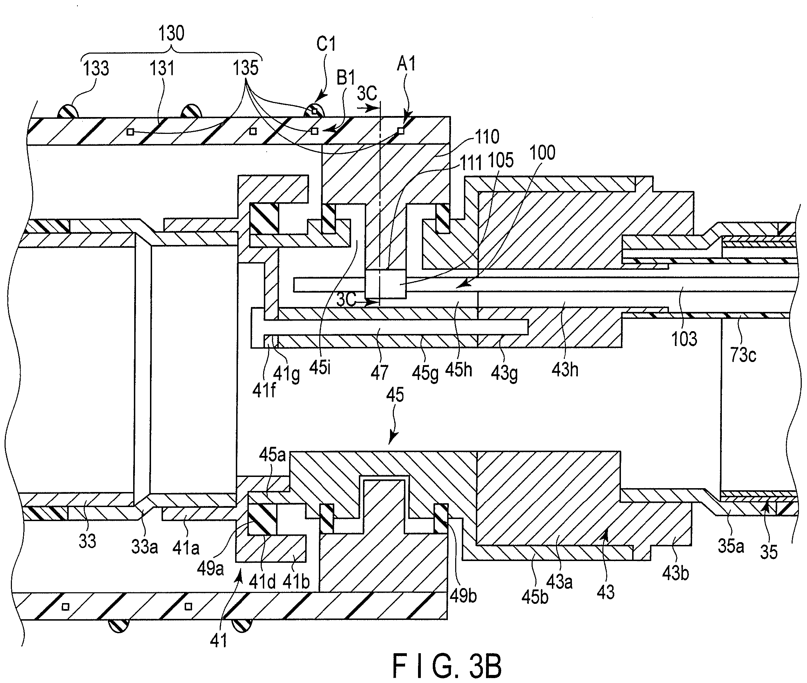

As shown in FIG. 3A and FIG. 3B, the proximal end portion of the bending section 33 is coupled with a bending section side mouth ring 33a. Further, as shown in FIG. 3A and FIG. 3B, the distal end portion of the flexible tube section 35 is coupled with a flexible tube section side mouth ring 35a.

### [Coupling Structure 40 of Proximal End Portion of Bending Section 33 and Distal End Portion of Flexible Tube Section 35]

As shown in FIG. 3A and FIG. 3B, a coupling structure 40 has a tubular mouth ring 41 that fits into a proximal end portion of the bending section side mouth ring 33a to assure water-tightness and a tubular mouth ring 43 that fits into the flexible tube section side mouth ring 35a to assure water-tightness. The tubular shape includes, e.g., a cylindrical shape. Additionally, the coupling structure 40 further has a cylindrical member 45 coupled with the mouth ring 41 and the mouth ring 43 in the longitudinal axis direction to assure water-tightness, and a coupling member 47, e.g., a pin that couples the mouth ring 41, the mouth ring 43, and the cylindrical member 45 with one another. The coupling structure 40 can be cleaned and sterilized.

### [Mouth Ring 41]

As shown in FIG. 3B, the mouth ring 41 has a distal end portion 41a into which the bending section side mouth ring 33a is inserted and fitted when the mouth ring 41 is coupled with the bending section side mouth ring 33a, and a proximal end portion 41b.

Furthermore, the mouth ring 41 also has a ring-like groove portion 41d that is arranged in an edge portion of the proximal end portion 41b and is concave from the proximal end portion 41b toward the distal end portion 41a, and a distal end portion 45a of the cylindrical member 45 is inserted to be fitted into the groove portion when the mouth ring 41 is coupled with the cylindrical member 45. The ring-like shape includes, e.g., an annular shape.

As shown in FIG. 3B, the distal end portion 45a of the cylindrical member 45 is inserted into the groove portion 41d. As shown in FIG. 3B, in this state, a water-tightness assuring member 49a such as an O-ring is arranged in the groove portion 41d. The water-tightness assuring member 49a is appressed against the proximal end portion 41b of the mouth ring 41 and the distal end portion 45a of the cylindrical member 45 to assure water-tightness between the mouth ring 41 and the cylindrical member 45.

Further, as shown in FIG. 3B, the mouth ring 41 has a tabular protruding portion 41f that is formed when part of a peripheral surface of the proximal end portion 41b protrudes toward the inside of the mouth ring 41. The protruding portion 41f has an engagement hole 41g that pierces through the protruding portion 41f in the longitudinal axis direction of the mouth ring 41. The coupling member 47 pierces through and engages with the engagement hole 41g when the proximal end portion of the bending section 33 and the distal end portion of the flexible tube section 35 are coupled with.

### [Mouth Ring 43]

As shown in FIG. 3B, the mouth ring 43 has a distal end portion 43a that is inserted and fitted into a proximal end portion 45b of the cylindrical member 45, and a proximal end portion 43b into which the flexible tube section side mouth ring 35a is inserted and fitted.

Additionally, as shown in FIG. 3B, the mouth ring 43 further has a concave portion 43g that is arranged on the distal end portion 43a side and arranged on the same straight line as the engagement hole 41g in the longitudinal axis direction to engage with the coupling member 47 when the proximal end portion of the bending section 33 and the distal end portion of the flexible tube section 35 are coupled with.

Further, as shown in FIG. 3B, the mouth ring 43 also has a through hole portion 43h that is arranged along the longitudinal axis direction of the mouth ring 43 and pierces through a thick wall portion of the mouth ring 43, and a later-described shaft member 103 is inserted into. The through hole portion 43h is arranged to deviate from the concave portion 43g in the radial direction of the mouth ring 43. The through hole portion 43h is arranged on, e.g., an outer side of the concave portion 43g.

### [Cylindrical Member 45]

As shown in FIG. 3B, the cylindrical member 45 has the distal end portion 45a that fits into the groove portion 41d of the mouth ring 41, and the proximal end portion 45b that fits into the distal end portion 43a of the mouth ring 43 while covering the distal end portion 43a of the mouth ring 43.

Further, as shown in FIG. 3B, the cylindrical member 45 has a holding hole 45g that holds the coupling member 47. The holding hole 45g is arranged in a thick wall portion of the cylindrical member 45 and pierces through the thick wall portion of the cylindrical member 45 in the longitudinal axis direction of the cylindrical member 45. The holding hole 45g is arranged on the same straight line as the engagement hole 41g and the concave portion 43g in the longitudinal axis direction and communicates with the engagement hole 41g and the concave portion 43g, when the proximal end portion of the bending section 33 and the distal end portion of the flexible tube section 35 are coupled with. The holding hole 45g holds the coupling member 47 when the coupling member 47 pierces through the holding hole portion 45g.

Furthermore, the cylindrical member 45 also has a gear arrangement section 45h where a distal end portion of the shaft member 103 and a later-described gear member 105 are arranged. The gear arrangement section 45h is formed as a hollow portion in which the distal end portion of the shaft member 103 and the gear member 105 are arranged. The gear arrangement section 45h is arranged in the thick wall portion of the tubular member 45 to communicate with the through hole portion 43h when the proximal end portion of the bending section 33 and the distal end portion of the flexible tube section 35 are coupled with, in the longitudinal axis direction. The gear arrangement section 45h is arranged to deviate from the holding hole 45g in the radial direction of the cylindrical member 45. The gear arrangement section 45h is arranged on, e.g., an outer side of the holding hole 45g.

Additionally, the cylindrical member 45 further has an opening portion 45i that is arranged in part of a peripheral surface of the cylindrical member 45 and communicates with the outside and the gear arrangement section 45h in the radial direction of the cylindrical member 45.

### [Example of Coupling of Proximal End Portion of Bending Section 33 and Distal End Portion of Flexible Tube Section 35]

### (Step 1)

The flexible tube section side mouth ring 35a is inserted and fitted into the proximal end portion 43b of the mouth ring 43.

### (Step 2)

Next, the distal end portion 43a of the mouth ring 43 is inserted into the proximal end portion 45b of the cylindrical member 45 so that the gear arrangement section 45h is arranged on the same straight line as the through hole portion 43h in the longitudinal axis direction and further communicates with the through hole portion 43h and so that the concave portion 43g is arranged on the same straight line as the holding hole 45g in the longitudinal axis direction and further communicates with the holding hole 45g. At this time, the distal end portion 43a of the mouth ring 43 is covered with the proximal end portion 45b of the cylindrical member 45 and fits into the proximal end portion 45b of the cylindrical member 45.

When the mouth ring 43 and the cylindrical member 45 fit into, the mouth ring 43 and the cylindrical member 45 assure water-tightness with each other.

### (Step 3)

The water-tightness assuring member 49a is arranged in the groove portion 41d.

Next, the distal end portion 45a of the cylindrical member 45 is inserted and fitted into the groove portion 41d so that the engagement hole 41g, the concave portion 43g and the holding hole 45g are mutually arranged on the same straight line in the longitudinal axis direction so that the engagement hole 41g communicates with the holding hole 45g.

At this time, the water-tightness assuring member 49a is appressed against the proximal end portion 41b of the mouth ring 41 and the distal end portion 45a of the cylindrical member 45 to assure the water-tightness between the mouth ring 41 and the cylindrical member 45.

### (Step 4)

Next, the coupling member 47 pierces through the engagement hole 41g and the holding hole 45g and engages with the concave portion 43g. As a result, the flexible tube section 35, the mouth ring 43, the cylindrical member 45 and the mouth ring 41 are coupled with one another. (Step 5)

Then, the bending section side mouth ring 33a is inserted and fitted into the distal end portion 41a of the mouth ring 41. As a result, the proximal end portion of the bending section 33 and the distal end portion of the flexible tube section 35 are coupled with each other.

### [Operating Section 70]

As shown in FIG. 1, the operating section 70 has the main body section 71 from which the flexible tube section 35 extends, a grip section 73 that is coupled with a proximal end portion of the main body section 71 and gripped by an operator who operates the endoscope 20, and a universal cord 75 connected to the grip section 73.

As shown in FIG. 1 and FIG. 2, the grip section 73 has the bending operating section 73a that operates the bending section 33 to bend, a drive member insertion opening 73b into which the later-described drive member 101 is inserted, and a rotary operating section 73d that operates a rotating direction of the later-described shaft member 103.

The bending operating section 73a is connected to a non-illustrated operation wire inserted into the grip section 73, the main body section 71, and the flexible tube section 35. A distal end portion of the operation wire is coupled with the distal end portion of the bending section 33. When the bending operating section 73a is operated, the operation wire is pulled. When the operation wire is pulled, the bending section 33 bends.

As shown in FIG. 1, the drive member insertion opening 73b is coupled with a proximal end portion of a shaft member insertion channel 73c. The drive member insertion opening 73b is an insertion opening through which the shaft member 103 is inserted into the shaft member insertion channel 73c. As shown in FIG. 1 and FIG. 3B, the shaft member insertion channel 73c is arranged from the grip section 73 to the flexible tube section 35 via the main body section 71 in the inserting section 30. Furthermore, a distal end portion of the shaft member insertion channel 73c is coupled with the mouth ring 43 to communicate with the through hole portion 43h.

As shown in FIG. 1 and FIG. 2, the rotary operating section 73d has a counterclockwise operating section 73e that operates the shaft member 103 so that the shaft member 103 rotates in a counterclockwise direction by a drive force of the drive member 101 and a clockwise operating section 73f that operates the shaft member 103 so that the shaft member 103 rotates in a clockwise direction by the drive force of the drive member 101. The counterclockwise operating section 73e and the clockwise operating section 73f are connected to the control unit 200 through the universal cord 75 and a connecting section 75a.

As shown in FIG. 1, the universal cord 75 has the connecting section 75a connected to the control unit 200 and the light source unit 220.

### [Configuration 2 of Endoscope 20]

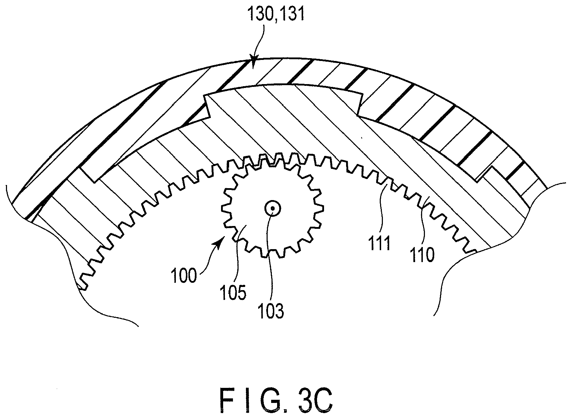

As shown in FIG. 1, FIG. 3B, and FIG. 3C, the endoscope 20 further has a rotary drive mechanism 100, a first rotary member 110, and a spiral rotary member 130. The rotary drive mechanism 100, the first rotary member 110 and the spiral rotary member 130 function as a propulsion assist unit which imparts, to the inserting section 30, a propulsive force to enable insertion of the inserting section 30 into a lumen or removal therefrom, thereby assisting in propulsion of the inserting section 30. The term propulsive force means an insertion force that acts on the inserting section 30 in the inserting direction of the inserting section 30 to assist the insertion of the inserting section 30, or a removal force that acts on the inserting section 30 in the removing direction of the inserting section 30 to assist the removal of the inserting section 30.

Moreover, for example, the rotary drive mechanism 100, the first rotary member 110 and the spiral rotary member 130 function as an insertion and removal assist unit that assists the insertion and the removal of the inserting section 30 into and from the lumen.

### [Rotary Drive Mechanism 100]

As shown in FIG. 1, FIG. 3B, and FIG. 3C, the rotary drive mechanism 100 is arranged in the inserting section 30. As shown in FIG. 1, FIG. 3B, and FIG. 3C, the rotary drive mechanism 100 has the drive member 101 that is connected to the control unit 200 through the cable 101a and inserted into the drive member insertion opening 73b, the shaft member 103 that has the distal end portion and a proximal end portion coupled with the drive member 101 and rotates in a periaxial direction of the longitudinal axis of the first rotary member 110 by the drive force of the drive member 101, and the gear member 105 that is arranged in the distal end portion of the shaft member 103 and functions as an outer peripheral tooth section.

The drive member 101 has, e.g., a motor. The drive member 101 has the drive force to rotate the first rotary member 110 around the longitudinal axis of the first rotary member 110.

As shown in FIG. 3B, the shaft member 103 is inserted into the shaft member insertion channel 73c. The shaft member 103 is arranged along the longitudinal axis direction. The shaft member 103 has, e.g., a flexible torque wire. The shaft member 103 rotates around the longitudinal axis of the shaft member 103 by the drive force of the drive member 101. The distal end portion of the shaft member 103 is arranged in the gear arrangement section 45h as shown in FIG. 3B.

As shown in FIG. 3B and FIG. 3C, the gear member 105 is arranged in the gear arrangement section 45h to mesh with a later-described inner peripheral tooth section 111 of the first rotary member 110. The gear member 105 is arranged in the distal end portion of the shaft member 103 so that the gear member 105 rotates around an axis of the gear member 105 in accordance with the rotation of the shaft member 103. Furthermore, the gear member 105 rotates around the axis of the gear member 105 in accordance with the rotation of the shaft member 103 in a state where the gear member 105 meshes with the first rotary member 110, thereby rotating the first rotary member 110.

The shaft member 103 and the gear member 105 are transmission rotary members that transmit the drive force of the drive member 101 to the first rotary member 110 and rotate the first rotary member 110 by the drive force.

### [First Rotary Member 110]

As shown in FIG. 3B, the first rotary member 110 is, e.g., a gear base member. The first rotary member 110 has, e.g., a tubular shape. The tubular shape includes, e.g., a cylindrical shape. As shown in FIG. 3B and FIG. 3C, the first rotary member 110 has the inner peripheral tooth section 111 that is fixed to an inner peripheral surface of the first rotary member 110 and meshes with the gear member 105. This inner peripheral tooth section 111 has a ring shape.

As shown in FIG. 3C, when the gear member 105 rotates around the axis of the gear member 105 in a state where the gear member 105 meshes with the inner peripheral tooth section 111, the first rotary member 110 rotates together with the inner peripheral tooth section 111 around the axis of the first rotary member 110. As described above, the first rotary member 110 is coupled with the rotary drive mechanism 100 and rotates around the axis of the first rotary member 110, i.e., around the longitudinal axis C by the drive force transmitted from the rotary drive mechanism 100.

As shown in FIG. 3B and FIG. 3C, the first rotary member 110 is arranged in such a manner that, e.g., the inner peripheral tooth section 111 is inserted into the opening portion 45i and arranged in the gear arrangement section 45h, the inner peripheral tooth section 111 meshes with the gear member 105 arranged in the gear arrangement section 45h, and an outer peripheral surface of the first rotary member 110 outwardly protrudes in the radial direction of the cylindrical member 45 beyond outer peripheral surfaces of the mouth rings 41 and 43 and an outer peripheral surface of the cylindrical member 45.

Furthermore, the first rotary member 110 is arranged to assure the water-tightness with the cylindrical member 45. Therefore, as shown in FIG. 3B, a water-tightness assuring member 49b such as an O-ring is interposed between the first rotary member 110 and the cylindrical member 45 in the radial direction of the cylindrical member 45.

### [Spiral Rotary Member 130]

As shown in FIG. 1 and FIG. 3B, the spiral rotary member 130 functions as an auxiliary insertion and removal device that is inserted into or removed from the lumen together with the inserting section 30 in a state where the inserting section 30 having the longitudinal axis C is inserted into the spiral rotary member 130, thereby assisting in the insertion and the removal of the inserting section 30 into and from the lumen. As shown in FIG. 1, FIG. 3B, and FIG. 4A, the spiral rotary member 130 has a tubular main body section 131 that allows the inserting section 30 to be inserted thereinto, is fixed to the outer peripheral surface of the first rotary member 110 and rotates around the longitudinal axis C in accordance with the rotation of the first rotary member 110; and a fin section 133 that is arranged on an outer peripheral surface of the main body section 131 and spirally arranged around the longitudinal axis C. In the main body section, the tubular shape includes, e.g., a cylindrical shape.

### [Main Body Section 131]

The main body section 131 is made of, e.g., a cleanable and sterilizable resin. The main body section 131 has, e.g., flexibility. This resin is, e.g., polyurethane. As shown in FIG. 1, for example, a distal end portion of the main body section 131 is detachably attached to, e.g., the bending section 33. As shown in FIG. 1 and FIG. 3B, e.g., a proximal end portion of the main body section 131 is detachably attached to the outer peripheral surface of the first rotary member 110 so that the main body section 131 rotates around the longitudinal axis C in accordance with the rotation of the first rotary member 110. In this way, the first rotary member 110 is also an attaching portion to attach the spiral rotary member 130 to the inserting section 30. The main body section 131 is detachably attachable to the inserting section 30.

### [Fin Section 133]

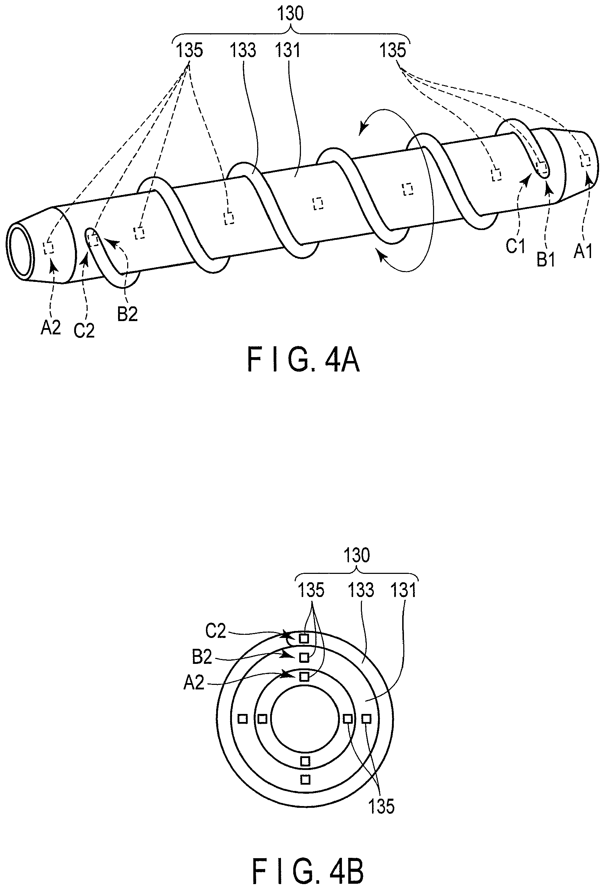

The fin section 133 is made of a resin such as a rubber that can be cleaned and sterilized. The fin section 133 is fixed to the outer peripheral surface of the main body section 131 by, e.g., bonding or welding. As shown in FIG. 1 and FIG. 4A, the fin section 133 is arranged to be spiral in, e.g., a clockwise fashion in a direction along which the distal end portion of the main body section 131 is seen from the proximal end portion of the main body section. The fin section 133 is projected and erected from the outer peripheral surface of the main body section 131 toward the outside.

As shown in FIG. 1 and FIG. 4A, the fin section 133 is shorter than the main body section 131 in the longitudinal axis direction of the spiral rotary member 130. Therefore, a distal end portion of the fin section 133 is arranged closer to a proximal end portion side of the main body section 131 to the distal end portion of the main body section 131, and a proximal end portion of the fin section 133 is arranged closer to a distal end portion side of the main body section 131 to the proximal end portion of the main body section 131. The fin section 133 is arranged to be spirally wound around the main body section 131.

As shown in FIG. 5B and FIG. 6B, for example, when the inserting section 30 is inserted into a lumen such as the pylorus 307 or the duodenum 309, the fin section 133 abuts on an inner wall of the lumen. In this state, when the main body section 131 rotates around the longitudinal axis C, the fin section 133 engages with the inner wall of the lumen, and the propulsive force acts on the inserting section 30 in the longitudinal axis direction. As a result, the inserting section 30 moves forward and backward in the lumen.

When the main body section 131 rotates in the clockwise direction, the insertion force acts on the inserting section 30, and insertability of the inserting section 30 is improved. Moreover, when the main body section 131 rotates in the counterclockwise direction, the removal force acts on the inserting section 30, and removability of the inserting section 30 is improved.

### [Main Body Section 131, Fin Section 133 and Inserting Section 30]

The main body section 131 and the fin section 133 are formed of a member that substantially transmits X-rays. Therefore, the main body section 131 and the fin section 133 are not clearly imaged by the X-ray irradiation imaging section 230. Consequently, as shown by dotted lines in FIG. 5B and FIG. 6B, the main body section 131 and the fin section 133 are formed as an unclearly imaged unclear region 141, and displayed as the unclear region 141 in the display section 240.

It is to be noted that in the inserting section 30 which is inserted into the spiral rotary member 130, the inserting section 30 is formed of a member that does not transmit X-rays. This member is, e.g., a metal. Therefore, the inserting section 30 is clearly imaged by the X-ray irradiation imaging section 230. In consequence, as shown by solid lines in FIG. 5B and FIG. 6B, the inserting section 30 is formed as a clearly imaged clear region 143, and displayed as the clear region 143 in the display section 240.

### [X-Ray Cutoff Section 135]

As shown in FIG. 1, FIG. 3B, FIG. 4A, and FIG. 4B, the spiral rotary member 130 further has an X-ray cutoff section 135 that cuts off X-rays. The X-ray cutoff section 135 is arranged in part of the main body section 131 in a peripheral direction of the main body section 131 so that a region to cut off X-rays and a region to substantially transmit X-rays are distinguished and formed in the periaxial direction of the longitudinal axis of the main body section 131. The X-ray cutoff section 135 is formed of a member that does not transmit X-rays. This member is, e.g., a metal. Therefore, the X-ray cutoff section 135 is clearly imaged by the X-ray irradiation imaging section 230. Consequently, as shown by solid lines in FIG. 5B and FIG. 6B, the X-ray cutoff section 135 is formed as the clearly imaged clear region 143, and displayed as the clear region 143 in the display section 240. Under X-ray observation, the X-ray cutoff section 135 functions as a distinguishing section that distinguishes the main body section 131 and the fin section 133 formed as the unclear region 141. In other words, the X-ray cutoff section 135 functions as an indicator section that indicates the main body section 131 and the fin section 133 that are unclearly imaged under X-ray observation. The X-ray cutoff section 135 is different from the main body section 131 and the fin section 133. The X-ray cutoff section 135 has, e.g., a solid shape.

As shown in FIG. 3B and FIG. 4A, the X-ray cutoff section 135 is arranged in at least a part of the spiral rotary member 130 in the longitudinal axis direction of the spiral rotary member 130. This point will be described hereinafter.

As shown in FIG. 3B and FIG. 4A, the X-ray cutoff section 135 is disposed in at least one of, e.g., a position A1 indicating the proximal end portion of the main body section 131, a position B1 indicating a proximal end portion side attaching portion of the main body section 131 to which the proximal end portion of the fin section 133 is attached, and a position C1 indicating the proximal end portion of the fin section 133. As shown in FIG. 3B, the X-ray cutoff section 135 is buried in, e.g., the proximal end portion of the main body section 131 at the position A1. This point substantially also applies to the position B1. The X-ray cutoff section 135 is buried in, e.g., the proximal end portion of the fin section 133 at the position C1. For the X-ray cutoff sections 135 disposed at the positions A1, B1, and C1, the respective X-ray cutoff sections 135 are different from one another. As shown in FIG. 3B, the X-ray cutoff section 135 disposed at the position A1 is arranged on the same straight line as the X-ray cutoff section 135 disposed at the position B1 in the longitudinal axis direction of the spiral rotary member 130. As shown in FIG. 3B, the X-ray cutoff section 135 disposed at the position B1 is arranged on the same straight line as the X-ray cutoff section 135 disposed at the position C1 in the radial direction of the spiral rotary member 130.

Further, as shown in FIG. 4A, the X-ray cutoff section 135 is disposed in at least one of, e.g., a position A2 indicating the distal end portion of the main body section 131, a position B2 indicating a distal end portion side attaching portion of the main body section 131 to which the distal end portion of the fin section 133 is attached, and a position C2 indicating the distal end portion of the fin section 133. The X-ray cutoff section 135 is buried in, e.g., the distal end portion of the main body section 131 at the position A2, similarly to the position A1. This point substantially also applies to the position B2. The X-ray cutoff section 135 is buried in, e.g., the distal end portion of the fin section 133 at the position C2. For the X-ray cutoff sections 135 disposed at the positions A2, B2, and C2, the respective X-ray cutoff sections 135 are different from one another. It is to be noted that a positional relation among the position A2, the position B2 and the position C2 is substantially the same as the positional relation among the position A1, the position B1 and the position C1 shown in FIG. 3B, and hence the positional relation is omitted in the drawing. Although not shown in the drawing, the X-ray cutoff section 135 disposed at the position A2 is arranged on the same straight line as the X-ray cutoff section 135 disposed at the position B2 in the longitudinal direction of the spiral rotary member 130, in the same manner as in the X-ray cutoff sections 135 disposed at the positions A1, B1 shown in FIG. 3B. Furthermore, although not shown in the drawing, the X-ray cutoff section 135 disposed at the position B2 is arranged on the same straight line as the X-ray cutoff section 135 disposed at the position C2 in the radial direction of the spiral rotary member 130, in the same manner as in the X-ray cutoff sections 135 disposed at the positions B1, C1 shown in FIG. 3B.

Further, as shown in FIG. 1, FIG. 3B and FIG. 4A, for example, the X-ray cutoff sections 135 are arranged along the longitudinal axis direction of the spiral rotary member 130. In this case, as shown in FIG. 3B and FIG. 4A, the X-ray cutoff sections 135 are buried in the main body section 131, and further interposed between the fin sections 133 in the longitudinal axis direction of the spiral rotary member 130. The X-ray cutoff sections 135 arranged in the main body section 131 are arranged on the same straight line along the longitudinal axis direction of the spiral rotary member 130, and are arranged via, e.g., an equal space from one another.

Further, as shown in FIG. 4B, for example, the X-ray cutoff sections 135 disposed at the positions A2, B2 and C2 are arranged on, e.g., the same straight line in the radial direction of the spiral rotary member 130, when the spiral rotary member 130 is seen from the front surface. This point also applies to the X-ray cutoff sections 135 disposed at the positions A1, B1, and C1.

Further, as shown in FIG. 1, FIG. 4B, FIG. 5B and FIG. 6B, the X-ray cutoff sections 135 to be arranged in the main body section 131 are arranged on, e.g., the same circumference in the periaxial direction of the longitudinal axis of the spiral rotary member 130. In this case, one of the X-ray cutoff sections 135 is disposed as much as a desirable angle, e.g., about 90 degrees away from the other of the X-ray cutoff sections 135 in the periaxial direction. The respective X-ray cutoff sections 135 are concentrically arranged around a central axis of the spiral rotary member 130.

In this way, as shown in FIG. 5B and FIG. 6B, the X-ray cutoff sections 135 are arranged so that the clear region 143 clearly imaged by X-rays and the unclear region 141 unclearly imaged by X-rays are distinguished in the peripheral direction of the spiral rotary member 130 under X-ray observation.

### [Control Unit 200]

As shown in FIG. 1, the control unit 200 has a control section 201 that controls driving of the drive member 101, the display section 210, and the light source unit 220 and a rotation speed input section 203 that inputs a rotation speed of the drive member 101, i.e., the spiral rotation member 130.

The control section 201 controls a rotating direction of the drive member 101 in accordance with an operation of the counterclockwise operating section 73e or the clockwise operating section 73f. Additionally, the control section 201 controls the rotation speed of the drive member 101 and controls the rotation speed of the spiral rotary member 130 based on an input amount of the rotation speed input section 203.

### [Function]

### [Position Confirmation of Spiral Rotary Member 130]

### [Position Confirmation when Distal End Portion of Spiral Rotary Member 130 is inserted into Pylorus]

The X-ray irradiation imaging section 230 irradiates the subject with X-rays. The inserting section 30 including the spiral rotary member 130 is inserted from a mouth 301 into a stomach 305 through an esophagus 303 as shown in FIG. 5A in the state where the subject is irradiated with X-rays. Furthermore, the inserting section 30 including the spiral rotary member 130 advances from the stomach 305 to the pylorus 307.

The subject including the lumen, the spiral rotary member 130 and the inserting section 30 are irradiated with X-rays and unclearly imaged by the X-ray irradiation imaging section 230. As a result, the unclear region 141 is formed. At this time, the X-ray cutoff section 135 cuts off X-rays, and is clearly imaged by the X-ray irradiation imaging section 230. The clear region 143 is formed. Further, as shown in FIG. 5B, the unclear region 141 shown by the dotted lines and the clear region 143 shown by the solid lines are displayed in the display section 240.

The X-ray cutoff section 135 is clearly displayed, and the position of the spiral rotary member 130 is thereby confirmed.

As shown in FIG. 5B, when the X-ray cutoff section 135 disposed at the position A2 is displayed in the display section 240, the position of the distal end portion of the main body section 131 is confirmed. As a result, the distal end portion of the main body section 131 does not abut on the stomach wall, but is smoothly guided to the pylorus 307 and smoothly inserted into the pylorus 307. Furthermore, it is confirmed that the distal end portion of the main body section 131 is inserted into the pylorus 307.

Further, as shown in FIG. 5B, when the X-ray cutoff section 135 disposed at the position B2 is displayed in the display section 240, there is confirmed a distal end portion side attaching position of the main body section 131 to which the distal end portion of the fin section 133 is attached. As a result, the distal end portion side attaching position is smoothly guided to the pylorus 307 and smoothly inserted into the pylorus 307. Furthermore, it is confirmed that the distal end portion side attaching position is inserted into the pylorus 307. That is, the distal end portion of the fin section 133 is smoothly guided to the pylorus 307 and smoothly inserted into the pylorus 307 indirectly through the main body section 131. Furthermore, it is confirmed indirectly through the main body section 131 that the distal end portion of the fin section 133 is inserted into the pylorus 307.

Further, as shown in FIG. 5B, when the X-ray cutoff section 135 disposed at the position C2 is displayed in the display section 240, the position of the distal end portion of the fin section 133 is confirmed. In consequence, the distal end portion of the fin section 133 is directly, securely and smoothly guided to the pylorus 307 and directly, securely and smoothly inserted into the pylorus 307. Furthermore, it is confirmed that the distal end portion of the fin section 133 is directly and securely inserted into the pylorus 307.

### [Position Confirmation of Entire Spiral Rotary Member 130]

Further, as shown in FIG. 5B and FIG. 6B, when the X-ray cutoff section 135 interposed between the fin sections 133 is displayed in the display section 240, the position of the entire spiral rotary member 130 is confirmed. As a result, the spiral rotary member 130 is smoothly guided to the pylorus 307 and smoothly inserted into the pylorus 307. It is also confirmed that the spiral rotary member 130 is inserted into the pylorus 307.

Additionally, as shown in FIG. 5B and FIG. 6B, one of the X-ray cutoff sections 135 is disposed about 90 degrees away from the other of the X-ray cutoff sections 135 in the periaxial direction. As a result, even if the X-ray cutoff section 135 is hidden behind the inserting section 30, at least one of the X-ray cutoff sections 135 is securely imaged and displayed, and the position of the entire spiral rotary member 130 is securely confirmed.

### [Position Confirmation when Proximal End Portion of Spiral Rotary Member 130 is inserted into Pylorus]

As shown in FIG. 6A, the spiral rotary member 130 is further inserted into the pylorus 307.

As shown in FIG. 6B, when the X-ray cutoff section 135 disposed at the position B1 is displayed in the display section 240, there is confirmed a proximal end portion side attaching position of the main body section 131 to which the proximal end portion of the fin section 133 is attached. As a result, the proximal end portion side attaching position is smoothly guided to the pylorus 307 and smoothly inserted into the pylorus 307. It is also confirmed that the proximal end portion side attaching position is inserted into the pylorus 307. That is, the proximal end portion of the fin section 133 is smoothly guided to the pylorus 307 and smoothly inserted into the pylorus 307 indirectly through the main body section 131. It is further confirmed through the main body section 131 that the proximal end portion of the fin section 133 is inserted into the pylorus 307. It is further confirmed through the main body section 131 that the entire fin section 133 is inserted into the pylorus 307.

Further, as shown in FIG. 6B, when the X-ray cutoff section 135 disposed at the position C1 is displayed in the display section 240, the position of the proximal end portion of the fin section 133 is confirmed. As a result, the proximal end portion of the fin section 133 is directly, securely and smoothly guided to the pylorus 307 and directly, securely and smoothly inserted into the pylorus 307. It is also confirmed that the proximal end portion of the fin section 133 is directly and more securely inserted into the pylorus 307. It is also directly and securely confirmed that the entire fin section 133 is inserted into the pylorus 307.

Additionally, as shown in FIG. 6B, when the X-ray cutoff section 135 disposed at the position A1 is displayed in the display section 240, the position of the proximal end portion of the main body section 131 is confirmed. As a result, the proximal end portion of the main body section 131 is smoothly guided to the pylorus 307 and smoothly inserted into the pylorus 307. It is also confirmed that the proximal end portion of the main body section 131 is inserted into the pylorus 307, and it is securely confirmed that the entire main body section 131, i.e., the entire spiral rotary member 130 is inserted into the pylorus 307.

### [Confirmation of Rotational situation of Spiral Rotary Member 130]

Confirmation of rotational situation of the spiral rotary member 130 is executed simultaneously with the abovementioned position confirmation of the spiral rotary member 130. Here, the X-ray cutoff section 135 is arranged in part of the main body section 131 in the peripheral direction so that the region to cut off X-rays and the region to substantially transmit X-rays are distinguished and formed in the periaxial direction of the longitudinal axis of the main body section 131. Therefore, the part of the main body section 131 in the peripheral direction is imaged and displayed. As a result, an operator can visually grasp that the main body section 131 rotates around the longitudinal axis and that the position of the imaged and displayed X-ray cutoff section 135 moves around the longitudinal axis of the main body section 131. In consequence, the operator can confirm that the main body section 131 rotates around the longitudinal axis.

### [Confirmation of Rotational Situation of Distal End Portion of Spiral Rotary Member 130]

When the clockwise operating section 73f and the rotation speed input section 203 are operated, the control section 201 controls the rotating direction of the drive member 101 so that the drive member 101 rotates in the clockwise direction, and controls the rotation speed of the drive member 101 based on the input amount of the rotation speed input section 203.

Furthermore, the shaft member 103 coupled with the drive member 101 and the gear member 105 arranged in the distal end portion of the shaft member 103 rotate around the longitudinal axis C in the clockwise direction. Consequently, the first rotation member 110 having the inner peripheral tooth section 111 that meshes with the gear member 105 and the main body section 131 attached to the first rotary member 110 rotate around the longitudinal axis C in the clockwise direction.

As the main body section 131 rotates, the X-ray cutoff section 135 also rotates. At this time, the X-ray cutoff section 135 cuts off X-rays and is clearly displayed in the display section 240. The X-ray cutoff section 135 is clearly displayed, and the rotational situation of the spiral rotary member 130 is thereby confirmed.

As shown in FIG. 5B, when the X-ray cutoff section 135 disposed at the position A2 is displayed in the display section 240, the rotational situation of the distal end portion of the main body section 131 is confirmed. Furthermore, even when the distal end portion of the main body section 131 is inserted into the pylorus 307 to receive resistance from the pylorus 307, the rotational situation of the distal end portion of the main body section 131 is confirmed.

Further, as shown in FIG. 5B, when the X-ray cutoff section 135 disposed at the position B2 is displayed in the display section 240, there is confirmed the rotational situation of the distal end portion side attaching position of the main body section 131 to which the distal end portion of the fin section 133 is attached. As a result, even when the distal end portion of the fin section 133 is inserted into the pylorus 307 to receive the resistance from the pylorus 307, the rotational situation of the distal end portion of the fin section 133 is confirmed indirectly through the main body section 131.

Additionally, as shown in FIG. 5B, when the X-ray cutoff section 135 disposed at the position C2 is displayed in the display section 240, the rotational situation of the distal end portion of the fin section 133 is confirmed. As a result, even when the distal end portion of the fin section 133 is inserted into the pylorus 307 to receive the resistance from the pylorus 307, the rotational situation of the distal end portion of the fin section 133 is directly and securely confirmed.

### [Confirmation of Rotational Situation of Entire Spiral Rotary Member 130]

Further, as shown in FIG. 5B and FIG. 6B, when the X-ray cutoff section 135 interposed between the fin sections 133 is displayed in the display section 240, the rotational situation of the entire spiral rotary member 130 is confirmed. As a result, a rotating portion of the spiral rotary member 130 and a non-rotating portion of the spiral rotary member 130 are confirmed. That is, it is confirmed which position of the spiral rotary member 130 rotates and which position of the spiral rotary member 130 is stopped.

Additionally, as shown in FIG. 5B and FIG. 6B, one of the X-ray cutoff sections 135 is disposed about 90 degrees away from the other of the X-ray cutoff sections 135 in the periaxial direction. As a result, even if the X-ray cutoff section 135 is hidden behind the inserting section 30, at least one of the X-ray cutoff sections 135 is securely imaged and displayed, and the rotational situation of the entire spiral rotary member 130 is securely confirmed.

### [Confirmation of Rotational Situation of Proximal End Portion of Spiral Rotary Member 130]

Furthermore, when the X-ray cutoff section 135 disposed at the position B1 is displayed in the display section 240, there is confirmed the rotational situation of the proximal end portion side attaching position of the main body section 131 to which the proximal end portion of the fin section 133 is attached. As a result, even when the proximal end portion of the fin section 133 is inserted into the pylorus 307 to receive the resistance from the pylorus 307, the rotational situation of the proximal end portion of the fin section 133 is confirmed indirectly through the main body section 131.

Furthermore, when the X-ray cutoff section 135 disposed at the position C1 is displayed in the display section 240, the rotational situation of the proximal end portion of the fin section 133 is confirmed. As a result, even when the proximal end portion of the fin section 133 is inserted into the pylorus 307 to receive the resistance from the pylorus 307, the rotational situation of the proximal end portion of the fin section 133 is directly and securely confirmed.

When the X-ray cutoff section 135 disposed at the position A1 is displayed in the display section 240, the rotational situation of the proximal end portion of the main body section 131 is confirmed. Furthermore, even when the proximal end portion of the main body section 131 is inserted into the pylorus 307 to receive the resistance from the pylorus 307, the rotational situation of the proximal end portion of the main body section 131 is confirmed.

It is to be noted that in the confirmation of the position and rotational situation of the spiral rotary member 130, the description has been given as to one example where the spiral rotary member 130 is inserted into the pylorus 307. This point substantially also applies to a case where the spiral rotary member 130 is removed from the pylorus 307, a case where the spiral rotary member 130 is inserted into or removed from the duodenum 309, a case where the spiral rotary member 130 is inserted into or removed from the cardiac orifice 311, and a case where the spiral rotary member 130 is inserted into or removed from the esophagus 303. That is, the spiral rotary member 130 is inserted into or removed from the lumen in the same manner as described above.

### [Effect]

As described above, in the present embodiment, the position and rotational situation of the spiral rotary member 130 that functions as the auxiliary insertion and removal device can easily be confirmed by the X-ray cutoff section 135 under X-ray observation.

Further, in the present embodiment, when the X-ray cutoff section 135 is disposed at the position A1, for example, it can be confirmed whether or not the proximal end portion of the main body section 131 is inserted into or removed from the lumen, and it can be confirmed whether or not the proximal end portion of the main body section 131 rotates. Further, in the present embodiment, for example, when the inserting section 30 passes the pylorus 307 from the stomach 305 to be inserted into the duodenum 309, it can securely be confirmed that the entire spiral rotary member 130 is inserted from the stomach 305 into the pylorus 307. Further, in the present embodiment, for example, when the inserting section 30 passes the cardiac orifice 311 from the stomach 305 to be removed from the esophagus 303, the proximal end portion of the main body section 131 does not abut on the stomach wall, but can smoothly be guided to the cardiac orifice 311 and can smoothly be inserted into the cardiac orifice 311. Further, in the present embodiment, it can be confirmed that the proximal end portion of the main body section 131 is inserted into the cardiac orifice 311. Further, in the present embodiment, the rotational situation of the proximal end portion of the main body section 131 can be confirmed simultaneously with the abovementioned position confirmation. As described above, in the present embodiment, the position and rotational situation of the proximal end portion of the main body section 131 can easily be confirmed.

Further, in the present embodiment, when the X-ray cutoff section 135 is disposed at the position B1, for example, it can be confirmed indirectly through the main body section 131 whether or not the proximal end portion of the fin section 133 is inserted into or removed from the lumen, and it can be confirmed indirectly through the main body section 131 whether or not the proximal end portion of the fin section 133 rotates. Further, in the present embodiment, for example, when the inserting section 30 passes the pylorus 307 from the stomach 305 to be inserted into the duodenum 309, it can be confirmed that the entire fin section 133 is inserted from the stomach 305 into the pylorus 307. Further, in the present embodiment, for example, when the inserting section 30 passes the cardiac orifice 311 from the stomach 305 to be removed from the esophagus 303, the proximal end portion of the fin section 133 does not abut on the stomach wall, but can smoothly be guided to the cardiac orifice 311 and can smoothly be inserted into the cardiac orifice 311. Further, in the present embodiment, it can indirectly be confirmed that the proximal end portion of the fin section 133 is inserted into the cardiac orifice 311. Further, in the present embodiment, the rotational situation of the proximal end portion of the fin section 133 can indirectly be confirmed simultaneously with the abovementioned position confirmation. As described above, in the present embodiment, the labor of arranging the X-ray cutoff section 135 in the thin fin section 133 can be saved. Furthermore, the position and rotational situation of the proximal end portion of the fin section 133 can easily be confirmed.

Further, in the present embodiment, when the X-ray cutoff section 135 is disposed at the position C1, for example, it can more directly be confirmed whether or not the proximal end portion of the fin section 133 is inserted into or removed from the lumen, and it can more directly be confirmed whether or not the proximal end portion of the fin section 133 rotates. Further, in the present embodiment, for example, when the inserting section 30 passes the pylorus 307 from the stomach 305 to be inserted into the duodenum 309, it can more directly be confirmed that the entire fin section 133 is inserted from the stomach 305 into the pylorus 307. Further, in the present embodiment, for example, when the inserting section 30 passes the cardiac orifice 311 from the stomach 305 to be removed from the esophagus 303, the proximal end portion of the fin section 133 does not abut on the stomach wall, but can smoothly and more directly be guided to the cardiac orifice 311 and can smoothly and more directly be inserted into the cardiac orifice 311. Further, in the present embodiment, it can more directly be confirmed that the proximal end portion of the fin section 133 is inserted into the cardiac orifice 311. Further, in the present embodiment, the rotational situation of the proximal end portion of the fin section 133 can directly be confirmed simultaneously with the abovementioned position confirmation. As described above, in the present embodiment, the position and rotational situation of the proximal end portion of the fin section 133 can easily and more directly be confirmed.

Further, in the present embodiment, when the X-ray cutoff section 135 is disposed at the position A2, for example, it can be confirmed whether or not the distal end portion of the main body section 131 is inserted into or removed from the lumen, and it can be confirmed that the distal end portion of the main body section 131 rotates. Further, in the present embodiment, for example, when the inserting section 30 passes the cardiac orifice 311 from the stomach 305 to be removed from the esophagus 303, it can securely be confirmed that the entire spiral rotary member 130 is inserted from the stomach 305 into the cardiac orifice 311. Further, in the present embodiment, for example, when the inserting section 30 passes the pylorus 307 from the stomach 305 to be inserted into the duodenum 309, the distal end portion of the main body section 131 does not abut on the stomach wall, but can smoothly be guided to the pylorus 307 and can smoothly be inserted into the pylorus 307. Further, in the present embodiment, it can be confirmed that the distal end portion of the main body section 131 is inserted into the pylorus 307. Further, in the present embodiment, the rotational situation of the distal end portion of the main body section 131 can be confirmed simultaneously with the abovementioned position confirmation. As described above, in the present embodiment, the position and rotational situation of the distal end portion of the main body section 131 can easily be confirmed.

Further, in the present embodiment, when the X-ray cutoff section 135 is disposed at the position B2, for example, it can be confirmed indirectly through the main body section 131 whether or not the distal end portion of the fin section 133 is inserted into or removed from the lumen, and it can be confirmed indirectly through the main body section 131 whether or not the distal end portion of the fin section 133 rotates. Further, in the present embodiment, for example, when the inserting section 30 passes the cardiac orifice 311 from the stomach 305 to be removed from the esophagus 303, it can be confirmed that the entire fin section 133 is inserted from the stomach 305 into the cardiac orifice 311. Further, in the present embodiment, for example, when the inserting section 30 passes the pylorus 307 from the stomach 305 to be inserted into the duodenum 309, the distal end portion of the fin section 133 does not abut on the stomach wall, but can smoothly be guided to the pylorus 307 and can smoothly be inserted into the pylorus 307. Further, in the present embodiment, it can indirectly be confirmed that the distal end portion of the fin section 133 is inserted into the pylorus 307. Further, in the present embodiment, the rotational situation of the fin section 133 can indirectly be confirmed simultaneously with the abovementioned position confirmation. As described above, in the present embodiment, the labor of arranging the X-ray cutoff section 135 in the thin fin section 133 can be saved. Furthermore, the position and rotational situation of the distal end portion of the fin section 133 can indirectly and easily be confirmed.

Further, in the present embodiment, when the X-ray cutoff section 135 is disposed at the position C2, for example, it can more directly be confirmed whether or not the distal end portion of the fin section 133 is inserted into or removed from the lumen, and it can more directly be confirmed whether or not the distal end portion of the fin section 133 rotates. Further, in the present embodiment, for example, when the inserting section 30 passes the cardiac orifice 311 from the stomach 305 to be removed from the esophagus 303, it can more directly be confirmed that the entire fin section 133 is inserted from the stomach 305 into the cardiac orifice 311. Further, in the present embodiment, for example, when the inserting section 30 passes the pylorus 307 from the stomach 305 to be inserted into the duodenum 309, the distal end portion of the fin section 133 does not abut on the stomach wall, but can smoothly be guided to the pylorus 307 and can smoothly be inserted into the pylorus 307. Further, in the present embodiment, it can directly be confirmed that the distal end portion of the fin section 133 is inserted into the pylorus 307. Further, in the present embodiment, the rotational situation of the fin section 133 can directly be confirmed simultaneously with the abovementioned position confirmation. As described above, in the present embodiment, the position and rotational situation of the distal end portion of the fin section 133 can easily and more directly be confirmed.

Further, in the present embodiment, when the X-ray cutoff section 135 is interposed between the fin sections 133 and imaged, the position and rotational situation of the entire spiral rotary member 130 can be confirmed. As a result, in the present embodiment, the spiral rotary member 130 can smoothly be guided to the pylorus 307, the spiral rotary member 130 can smoothly be inserted into the pylorus 307, and it can be confirmed that the spiral rotary member 130 is inserted into the pylorus 307. Further, in the present embodiment, the rotating portion of the spiral rotary member 130 and the non-rotating portion of the spiral rotary member 130 can be confirmed. That is, it can be confirmed which position of the spiral rotary member 130 rotates and which position of the spiral rotary member 130 is stopped.

Further, in the present embodiment, one of the X-ray cutoff sections 135 is disposed about 90 degrees away from the other of the X-ray cutoff sections 135 in the periaxial direction. As a result, in the present embodiment, even if the X-ray cutoff section 135 is hidden behind the inserting section 30, at least one of the X-ray cutoff sections 135 can securely be imaged, and the situation of the entire spiral rotary member 130 can securely be confirmed.

It is to be noted that the X-ray cutoff sections 135 are arranged on the same straight line along the longitudinal axis direction of the spiral rotary member 130, but the present invention does not have to be restricted thereto. The X-ray cutoff section 135 may be arranged in the form of a band in the longitudinal axis direction of the spiral rotary member 130. In this case, the distal end portion of the X-ray cutoff section 135 may be positioned at one of the position A2, the position B2 and the position C2, and the proximal end portion of the X-ray cutoff section 135 positioned at one of the position A1, the position B1 and the position C1 can suffice.

Furthermore, the main body section 131 having a tubular shape can suffice.

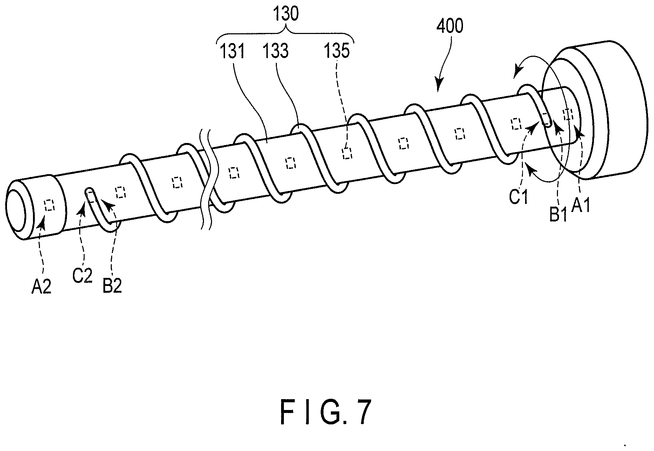

Further, in the present embodiment, the spiral rotary member 130 functions as the auxiliary insertion and removal device, but the present invention does not have to be restricted thereto. As shown in FIG. 7, an overtube 400 into which the inserting section 30 of the endoscope 20 is inserted may function as the auxiliary insertion and removal device. The overtube 400 has the spiral rotary member 130, and the spiral rotary member 130 has the main body section 131, the fin section 133 and the X-ray cutoff section 135. The X-ray cutoff section 135 is arranged as described above.

### (Second Embodiment)

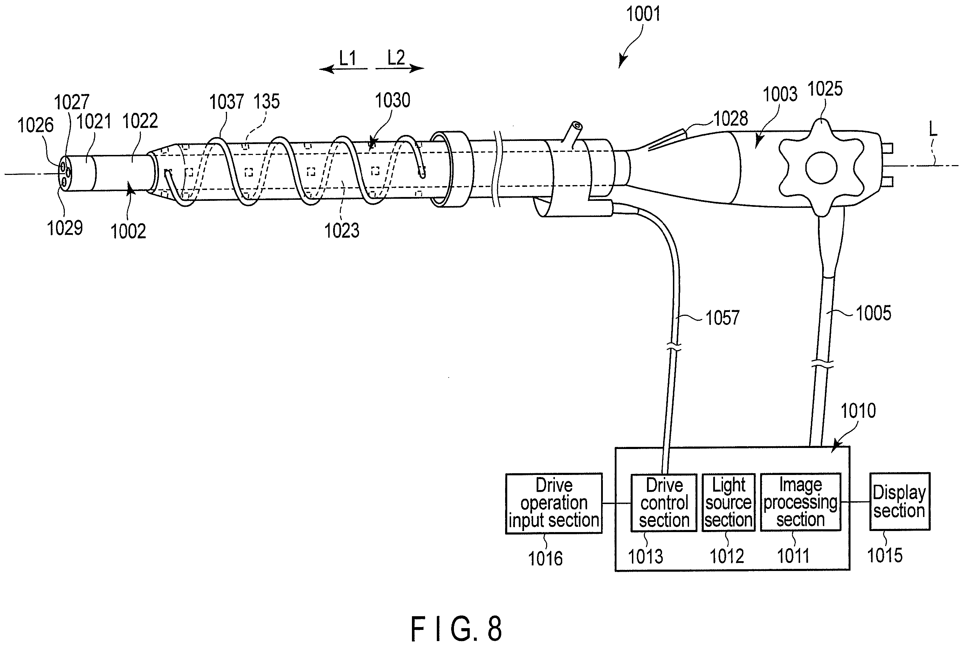

A second embodiment of the present invention will be described with reference to FIG. 8 to FIG. 13. FIG. 8 is a view showing an endoscope device 1001 which is an insertion device of the present embodiment. As shown in FIG. 8, the endoscope device 1001 has a longitudinal axis L. One of directions parallel to the longitudinal axis L is a distal end direction (a direction of an arrow L1 of FIG. 8), and an opposite direction to the distal end direction is a proximal end direction (a direction of an arrow L2 of FIG. 8). As shown in FIG. 8, the endoscope device 1001 comprises an inserting section (an endoscope inserting section) 1002 extending along the longitudinal axis L from the proximal end direction toward the distal end direction, and an operating section (an endoscope operating section) 1003 arranged on a proximal end direction side to the inserting section 1002. The inserting section 1002 is inserted into a body cavity during use of the endoscope device 1001.

The operating section 1003 is connected to one end of a universal cable 1005. The other end of the universal cable 1005 is connected to a control unit 1010. The control unit 1010 comprises an image processing section 1011, a light source section 1012, and a drive control section 1013. The image processing section 1011 of the control unit 1010 is electrically connected to a display section 1015 such as a monitor. Furthermore, the drive control section 1013 of the control unit 1010 is electrically connected to a drive operation input section 1016 such as a foot switch.

The inserting section 1002 comprises a distal end hard section 1021, a bending section 1022 arranged on the proximal end direct side to the distal end hard section 1021, and a flexible tube section 1023 arranged on the proximal end direct side to the bending section 1022. Furthermore, the operating section 1003 has a bending operation knob 1025 as a bending operation input section into which a bending operation to bend the bending section 1022 is input. In the inserting section 1002, a bending wire (not shown) extends along the longitudinal axis L. In the operating section 1003, a proximal end of the bending wire is connected to a pulley (not shown) coupled with the bending operation knob 1025. A distal end of the bending wire is connected to a distal end portion of the bending section 1022. By the bending operation of the bending operation knob 1025, the bending wire moves along the longitudinal axis L, and the bending section 1022 bends.

In the distal end hard section 1021, an imaging element (not shown) is disposed. The imaging element images a subject through an observation window 1026 disposed in the distal end hard section 1021. The imaging element is connected to one end of an imaging cable (not shown). The imaging cable passes through the inserting section 1002, the operating section 1003, and the universal cable 1005, and the other end of the imaging cable is connected to the image processing section 1011 of the control unit 1010. A subject image acquired by the imaging element is converted into an electric signal, and transmitted to the image processing section 1011 through the imaging cable. Furthermore, image processing is performed by the image processing section 1011, and the subject image is displayed in the display section 1015. Furthermore, in the inserting section 1002, a light guide (not shown) is extended. One end of the light guide passes through the operating section 1003 and the universal cable 1005, to be connected to the light source section 1012. Light emitted from the light source section 1012 is guided by the light guide, and the subject is irradiated with the light from an illumination window 1027 of the distal end hard section 1021.

In the inserting section 1002, a treatment tool channel tube (not shown) is extended. Further, on an outer surface of the operating section 1003, a treatment tool inserting section 1028 is disposed into which a treatment tool such as forceps is inserted. The treatment tool channel tube (not shown) passes through the operating section 1003, and one end of the treatment tool channel tube is connected to the treatment tool inserting section 1028. The treatment tool inserted from the treatment tool inserting section 1028 passes through the treatment tool channel tube to protrude from a treatment tool protrusion port 1029 of the distal end hard section 1021 toward the distal end direction. Further, in a state where the treatment tool is projected from the treatment tool protrusion port 1029 of the distal end hard section 1021, a treatment by the treatment tool is performed.

A spiral rotary member 1030 that functions as an auxiliary insertion and removal device is attached to the flexible tube section 1023 of the inserting section 1002. The spiral rotary member 1030 extends along the longitudinal axis L in an outer peripheral direction of the inserting section 1002. Furthermore, the inserting section 1002 is inserted into the spiral rotary member 1030. A larger part of the flexible tube section 1023 which is parallel to the longitudinal axis L is covered with the spiral rotary member 1030.

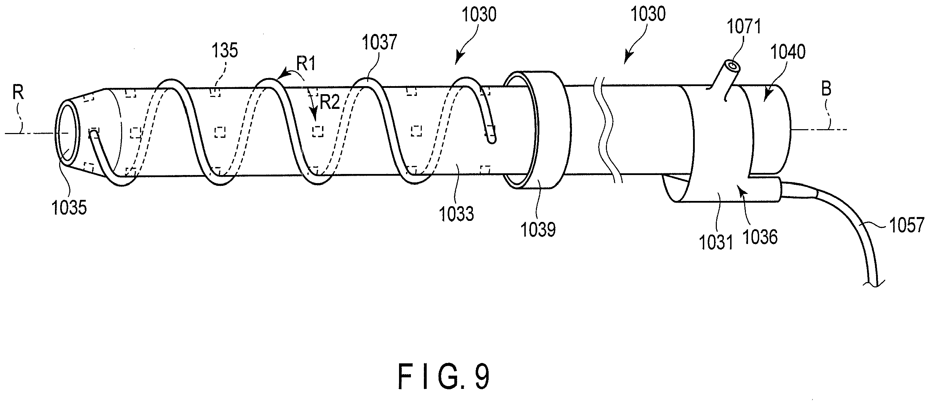

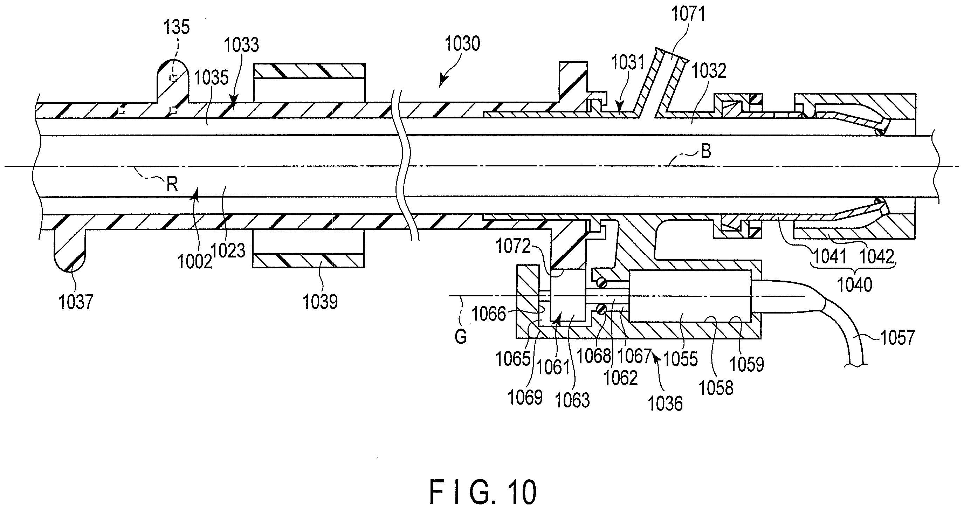

FIG. 9 and FIG. 10 are views showing a configuration of the spiral rotary member 1030. It is to be noted that FIG. 10 shows that the spiral rotary member 1030 is attached to the inserting section 1002. As shown in FIG. 9 and FIG. 10, the spiral rotary member 1030 comprises a drive source unit 1036 which a tubular main body 1031 is disposed. The tubular main body 1031 is made of a hard material, and does not bend due to an external force. Furthermore, the tubular main body 1031 has a main body axis B, and a main body hollow 1032 is formed on an inner peripheral direction side of the tubular main body 1031. The inserting section 1002 passes through the main body hollow 1032 to be inserted into the spiral rotary member 1030. In the main body hollow 1032 in a state where the spiral rotary member 1030 is attached to the inserting section 1002, the main body axis B of the tubular main body 1031 is the same axis as the longitudinal axis L.

An elongated rotary tubular section 1033 as a rotation target is attached to the drive source unit 1036. The rotary tubular section 1033 has about the same configuration as in the main body section 131. The rotary tubular section 1033 is attached to a distal end direction side of the tubular main body 1031. The rotary tubular section 1033 is made of a soft material and has flexibility. Therefore, the rotary tubular section 1033 bends in accordance with the bending of the inserting section 1002 by the external force. Furthermore, the rotary tubular section 1033 has a rotation axis R, and a tubular section hollow 1035 is formed on the inner peripheral direction side of the rotary tubular section 1033. The inserting section 1002 passes through the tubular section hollow 1035 to be inserted into the spiral rotary member 1030. In the tubular section hollow 1035 in the state where the spiral rotary member 1030 is attached to the inserting section 1002, the rotation axis R of the rotary tubular section 1033 is the same axis as the longitudinal axis L. Further, in the present embodiment, the main body axis B of the tubular main body 1031 is the same axis as the rotation axis R of the rotary tubular section 1033. The rotary tubular section 1033 can rotate to the tubular main body 1031 in directions around the rotation axis. Here, one of the directions around the rotation axis is a first rotating direction (a direction of an arrow R1 of FIG. 9), and an opposite direction to the first rotating direction is a second rotating direction (a direction of an arrow R2 of FIG. 9).

In an outer peripheral portion of the rotary tubular section 1033, a spiral fin section 1037 protruding toward the outer peripheral direction is disposed. The spiral fin section 1037 has about the same configuration as in the fin section 133. The spiral fin section 1037 is positioned in a distal end portion of the rotary tubular section 1033. Furthermore, the spiral fin section 1037 spirally extends along the rotation axis R (the longitudinal axis L). The spiral fin section 1037 is positioned on a first rotating direction side from the proximal end direction toward the distal end direction.

In the rotary tubular section 1033 and the spiral fin section 1037, X-ray cutoff sections 135 are arranged in the same manner as in the first embodiment.

Furthermore, on an outer peripheral direction side of the rotary tubular section 1033, a holding tube 1039 is disposed. The rotary tubular section 1033 is inserted into the holding tube 1039. The holding tube 1039 is positioned on the proximal end direction side to the spiral fin section 1037. Furthermore, the holding tube 1039 can rotate to the rotary tubular section 1033 in a periaxial direction of the rotation axis (a periaxial direction of the longitudinal axis).

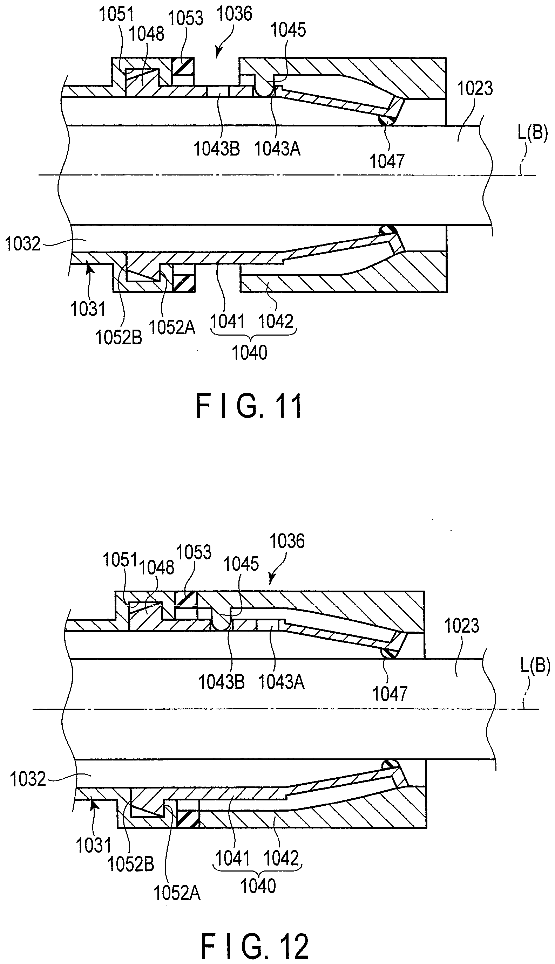

A fixing tubular section 1040 is attached to the proximal end direction side of the tubular main body 1031. The fixing tubular section 1040 is a part of the drive source unit 1036. The fixing tubular section 1040 is made of a hard material and does not bend by the external force. FIG. 11 and FIG. 12 are views showing configurations of the tubular main body 1031 and the fixing tubular section 1040. As shown in FIG. 11 and FIG. 12, the fixing tubular section 1040 comprises a proximal end side tubular member 1041 and a fastening member 1042 attached to the proximal end side tubular member 1041. In the proximal end side tubular member 1041, a first engagement hole 1043A and a second engagement hole 1043B are arranged. The second engagement hole 1043B is positioned on the distal end direction side to the first engagement hole 1043A.

In the fastening member 1042, an engaging protrusion 1045 is disposed. When the engaging protrusion 1045 engages with the first engagement hole 1043A or the second engagement hole 1043B, the fastening member 1042 is fixedly attached to the proximal end side tubular member 1041. In a state where the engaging protrusion 1045 is not engaged with the first engagement hole 1043A and the second engagement hole 1043B, the fastening member 1042 can move to the proximal end side tubular member 1041 along the longitudinal axis L. Here, FIG. 11 shows a state where the engaging protrusion 1045 is engaged with the first engagement hole 1043A, and FIG. 12 shows a state where the engaging protrusion 1045 is engaged with the second engagement hole 1043B.

On the inner peripheral direction side of the proximal end side tubular member 1041 of the fixing tubular section 1040, a ring-like elastic member 1047 is disposed. In the state where the spiral rotary member 1030 is attached to the inserting section 1002, the elastic member 1047 is positioned between the proximal end side tubular member 1041 of the fixing tubular section 1040 and an outer peripheral portion of the inserting section 1002 (the flexible tube section 1023) in a radial direction. In the state where the engaging protrusion 1045 is engaged with the first engagement hole 1043A or the second engagement hole 1043B, a pressing force acts from the fastening member 1042 to the proximal end side tubular member 1041 in an inner peripheral direction. When the pressing force acts on the proximal end side tubular member 1041, the elastic member 1047 contracts, and the elastic member 1047 is appressed against the proximal end side tubular member 1041 and the outer peripheral portion of the inserting section 1002. As a result, movement of the proximal end side tubular member 1041 to the inserting section 1002 in the direction parallel to the longitudinal axis L is regulated, and rotation of the proximal end side tubular member 1041 to the inserting section 1002 in the periaxial direction of the longitudinal axis (the periaxial direction of the rotation axis) is regulated. That is, the proximal end side tubular member 1041 is fixed to the inserting section 1002.

Furthermore, in the state where the engaging protrusion 1045 is engaged with the first engagement hole 1043A or the second engagement hole 1043B, the fastening member 1042 is fixed to the proximal end side tubular member 1041. Therefore, when the engaging protrusion 1045 engages with the first engagement hole 1043A or the second engagement hole 1043B, the fixing tubular section 1040 is fixed to the inserting section 1002.

In a distal end portion of the proximal end side tubular member 1041 of the fixing tubular section 1040, an engaging pawl 1048 protruding toward the outer peripheral direction is disposed. The engaging pawl 1048 is disposed over the whole periphery in the periaxial direction of the longitudinal axis. In a proximal end portion of the tubular main body 1031, an engagement groove 1051 is disposed. The engagement groove 1051 is disposed over the whole periphery in the periaxial direction of the longitudinal axis (a periaxial direction of the main body axis). When the engaging pawl 1048 is inserted into the engagement groove 1051 to engage therewith, the proximal end side tubular member 1041 of the fixing tubular section 1040 is attached to the tubular main body 1031.

In the engaging pawl 1048 of the proximal end side tubular member 1041, there are arranged abutment portions 1052A, 1052B that abut on the tubular main body 1031 in the engagement groove 1051. When the tubular main body 1031 abuts on the abutment portion 1052A of the engaging pawl 1048 in the engagement groove 1051, the movement of the tubular main body 1031 in the distal end direction is regulated. Furthermore, when the tubular main body 1031 abuts on the abutment portion 1052B of the engaging pawl 1048 in the engagement groove 1051, the movement of the tubular main body 1031 in the proximal end direction is regulated. That is, by the abutment portions 1052A, 1052B of the engaging pawl 1048, the movement of the tubular main body 1031 to the fixing tubular section 1040 in the direction parallel to the main body axis B (the longitudinal axis L) is regulated.

Here, in the state where the spiral rotary member 1030 is attached to the inserting section 1002, the fixing tubular section 1040 is fixed to the inserting section 1002 by the elastic member 1047. Therefore, in the state where the spiral rotary member 1030 is attached to the inserting section 1002, the movement of the tubular main body 1031 to the inserting section 1002 in the direction parallel to the main body axis B (the longitudinal axis L) is regulated. As described above, the elastic member 1047 and the abutment portions 1052A, 1052B function as a movement regulating section that regulates the movement of the tubular main body 1031 to the inserting section 1002 in the direction parallel to the main body axis B (the longitudinal axis L). It is to be noted that the rotation of the tubular main body 1031 in the periaxial direction of the main body axis (the periaxial direction of the longitudinal axis) is not regulated by the engaging pawl 1048. Therefore, the engagement groove 1051 can move to the engaging pawl 1048 in the periaxial direction of the main body axis (the periaxial direction of the rotation axis).

A ring-like elastic section 1053 is fixed to the tubular main body 1031. The elastic section 1053 is positioned between the tubular main body 1031 and the fastening member 1042 of the fixing tubular section 1040. In the state where the engaging protrusion 1045 is engaged with the first engagement hole 1043A (see FIG. 11), the fastening member 1042 is not in contact with the elastic section 1053. The fixing tubular section 1040 does not come in contact with the elastic section 1053, hence the tubular main body 1031 can rotate to the fixing tubular section 1040 in the periaxial direction of the rotation axis (the periaxial direction of the main body axis). As described above, in the state where the spiral rotary member 1030 is attached to the inserting section 1002, the fixing tubular section 1040 is fixed to the inserting section 1002. Therefore, in the state where the engaging protrusion 1045 is engaged with the first engagement hole 1043A, there is obtained a non-regulated state where the rotation of the tubular main body 1031 to the inserting section 1002 in the periaxial direction of the rotation axis is not regulated. In the non-regulated state, the inserting section 1002 and the fixing tubular section 1040 can rotate to the tubular main body 1031 in the periaxial direction of the longitudinal axis (the periaxial direction of the rotation axis).

On the other hand, in the state where the engaging protrusion 1045 is engaged with the second engagement hole 1043B (see FIG. 12), the fastening member 1042 abuts on the elastic section 1053. When the fastening member 1042 of the fixing tubular section 1040 abuts on the elastic section 1053, a friction force is generated between the elastic section 1053 and the fastening member 1042. Due to this friction force, the rotation of the tubular main body 1031 to the fixing tubular section 1040 in the periaxial direction of the rotation axis (the periaxial direction of the main body axis) is regulated. As described above, in the state where the spiral rotary member 1030 is attached to the inserting section 1002, the fixing tubular section 1040 is fixed to the inserting section 1002 by the elastic member 1047. Therefore, in the state where the engaging protrusion 1045 is engaged with the second engagement hole 1043B, there is obtained a regulated state where the rotation of the tubular main body 1031 to the inserting section 1002 in the periaxial direction of the rotation axis is regulated. In the regulated state, the rotation of the tubular main body 1031 to the inserting section 1002 in the periaxial direction of the rotation axis is regulated, hence the tubular main body 1031 is fixed to the inserting section 1002. As described above, the elastic member 1047 and the elastic section 1053 function as a rotation regulating section that regulates the rotation of the tubular main body 1031 to the inserting section 1002 in the periaxial direction of the rotation axis, in the state where the spiral rotary member 1030 is attached to the inserting section 1002.

Furthermore, in the non-regulated state where the engaging protrusion 1045 is engaged with the first engagement hole 1043A, the tubular main body 1031 is switched to a state where the tubular main body 1031 is rotatable to the fixing tubular section 1040 in the periaxial direction of the rotation axis. On the other hand, in the regulated state where the engaging protrusion 1045 is engaged with the second engagement hole 1043B, the tubular main body 1031 is switched to a state where the tubular main body 1031 is not rotatable to the fixing tubular section 1040 in the periaxial direction of the rotation axis. That is, the first engagement hole 1043A, the second engagement hole 1043B and the engaging protrusion 1045 function as a regulation switching section that switches the rotation regulating section (1047, 1053) to the non-regulated state where the rotation of the tubular main body 1031 to the inserting section 1002 in the periaxial direction of the rotation axis is not regulated and the regulated state where the rotation of the tubular main body 1031 to the inserting section 1002 in the periaxial direction of the rotation axis is regulated, in the state where the spiral rotary member 1030 is attached to the inserting section 1002.

As shown in FIG. 10, a motor 1055 as a drive member is attached to the tubular main body 1031. The motor 1055 is a part of the drive source unit 1036. The motor 1055 is received in a member hollow portion 1058. The member hollow portion 1058 is defined by a member hollow defining section 1059 of the tubular main body 1031. One end of a motor cable (a cable) 1057 is connected to the motor 1055. As shown in FIG. 8, the other end of the motor cable 1057 is connected to the drive control section 1013 of the control unit 1010. By the drive control section 1013, power is supplied to the motor 1055 through the motor cable 1057. When the power is supplied, the motor 1055 is driven. When the motor 1055 is driven, a rotational drive force to rotate the rotary tubular section 1033 in the periaxial direction of the rotation axis is produced.

As shown in FIG. 10, a drive force transmitting section 1061 is attached to the tubular main body 1031. The drive force transmitting section 1061 is a part of the drive source unit 1036. The drive force transmitting section 1061 is coupled with the motor 1055 through a shaft member 1062. The drive force transmitting section 1061 comprises a drive gear 1063. The drive gear 1063 has a gear axis G. When the motor 1055 is driven, the drive gear 1063 rotates in a periaxial direction of the gear axis. The drive force transmitting section 1061 is disposed in a gear hollow portion 1065. The gear hollow portion 1065 is defined by a gear hollow defining section 1066 of the tubular main body 1031.

A communication passage 1067 is formed between the gear hollow portion 1065 and the member hollow portion 1058. In the communication passage 1067, a seal ring 1068 is disposed. The seal ring 1068 maintains liquid-tightness between the shaft member 1062 and the tubular main body 1031. As a result, liquid is prevented from flowing from the gear hollow portion 1065 into the member hollow portion 1058. Furthermore, in the tubular main body 1031, there is disposed a covering section 1069 that covers an outer peripheral direction side of the drive force transmitting section 1061 (the drive gear 1063). The covering section 1069 prevents the drive force transmitting section 1061 from being exposed to the outside. Furthermore, in the tubular main body 1031, a pouring port 1071 piercing through the tubular main body 1031 in the radial direction is disposed. A lubricant is poured through the pouring port 1071.

In a proximal end portion of the rotary tubular section 1033, a rotation side gear section 1072 that meshes with the drive gear 1063 is disposed. When the drive gear 1063 rotates in the periaxial direction of the gear axis, the rotary drive force produced by driving of the motor 1055 is transmitted to the rotary tubular section 1033. The rotation side gear section 1072 receives the rotational drive force from the drive gear 1063 of the drive force transmitting section 1061. That is, the rotation side gear section 1072 is a drive force receiving section that receives the rotational drive force from the drive force transmitting section 1061. When the rotary drive force is transmitted, the rotary tubular section 1033 rotates to the tubular main body 1031 in the periaxial direction of the rotation axis. Here, in the regulated state where the rotation of the tubular main body 1031 to the inserting section 1002 in the periaxial direction of the rotation axis is regulated, when the rotary drive force is transmitted, the rotary tubular section 1033 rotates to the inserting section 1002 in the periaxial direction of the rotation axis (the periaxial direction of the longitudinal axis).

Next, operations and effects of the endoscope device 1001 and the spiral rotary member 1030 of the present embodiment will be described. During use of the endoscope device 1001, the inserting section 1002 to which the spiral rotary member 1030 is attached is inserted into a lumen. In the spiral rotary member 1030 in a state where the inserting section 1002 is inserted into the lumen, only the rotary tubular section 1033 is positioned in the lumen, and the tubular main body 1031 and the fixing tubular section 1040 are positioned outside of a body. Further, in this case, the engaging protrusion 1045 of the fastening member 1042 disposed in the fixing tubular section 1040 engages with the second engagement hole 1043B of the proximal end side tubular member 1041. In consequence, there is obtained the regulated state where the rotation of the tubular main body 1031 to the inserting section 1002 in the periaxial direction of the rotation axis (the periaxial direction of the longitudinal axis) is regulated. Further, in the state where the spiral rotary member 1030 is attached to the inserting section 1002, the movement of the tubular main body 1031 to the inserting section 1002 in the direction parallel to the main body axis B (the longitudinal axis L) is regulated.

Furthermore, the motor 1055 is driven by an operation in the drive operation input section 1016. In consequence, the drive gear 1063 of the drive force transmitting section 1061 rotates around the gear axis G, and the rotational drive force is transmitted to the rotary tubular section 1033. As a result, the rotary tubular section 1033 rotates to the inserting section 1002, the tubular main body 1031 and the fixing tubular section 1040 in the periaxial direction of the rotation axis (the periaxial direction of the longitudinal axis). Here, in the present embodiment, when the drive operation input section 1016, e.g., a foot switch is pressed, the motor 1055 is driven, and the rotary tubular section 1033 rotates. Consequently, when the inserting section 1002 and the spiral rotary member 1030 are moved in the direction parallel to the longitudinal axis L, an operator can perform a rotating operation of the rotary tubular section 1033 while holding the inserting section 1002. Therefore, when the inserting section 1002 and the spiral rotary member 1030 are moved in the direction parallel to the longitudinal axis L, use of hands and labor can be saved.

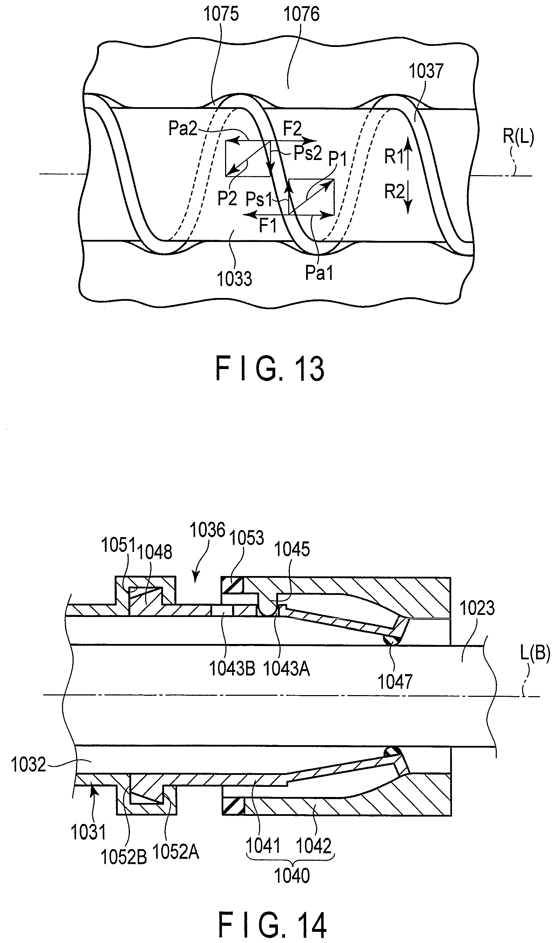

FIG. 13 is a view to explain a state where the rotary tubular section 1033 of the spiral rotary member 1030 rotates in the periaxial direction of the rotation axis in a lumen 1075. As shown in FIG. 13, in the lumen 1075, the spiral fin section 1037 of the rotary tubular section 1033 is in contact with a luminal paries 1076. Therefore, the distal end portion of the rotary tubular section 1033 has a pressed state where the pressing force acts on the spiral fin section 1037 from the luminal paries 1076 in the inner peripheral direction. When the rotary tubular section 1033 in the pressed state is rotated in a first rotating direction (a direction of an arrow R1 of FIG. 13) as one of the directions around the rotation axis, pressing force P1 acts from the spiral fin section 1037 onto the luminal paries 1076. The pressing force P1 is decomposed into a peripheral direction force component Ps1 in the first rotating direction and an axial direction force component Pa1 in the proximal end direction. As a reaction to the axial direction force component Pa1 of the pressing force P1, propulsive force F1 in the distal end direction acts from the luminal paries 1076 onto the inserting section 1002 and the spiral rotary member 1030. By the propulsive force F1, movability of the inserting section 1002 in the distal end direction parallel to the longitudinal axis L is improved in the lumen 1075.

On the other hand, when the rotary tubular section 1033 in the pressed state is rotated in a second rotating direction (a direction of an arrow R2 of FIG. 13) as an opposite direction to the first rotating direction, pressing force P2 acts from the spiral fin section 1037 onto the luminal paries 1076. The pressing force P2 is decomposed into a peripheral direction force component Ps2 in the second rotating direction and an axial direction force component Pa2 in the distal end direction. As a reaction to the axial direction force component Pa2 of the pressing force P2, propulsive force F2 acts from the luminal paries 1076 onto the inserting section 1002 and the spiral rotary member 1030 in the proximal end direction. By the propulsive force F2, the movability of the inserting section 1002 in the proximal end direction parallel to the longitudinal axis L is improved in the lumen 1075.

In the state where the spiral rotary member 1030 is attached to the inserting section 1002, movement of the tubular main body 1031 to the inserting section 1002 in the direction parallel to the main body axis B (the longitudinal axis L) is regulated. Therefore, the propulsive force F1 transmitted from the rotary tubular section 1033 to the tubular main body 1031 is suitably transmitted to the inserting section 1002 through the fixing tubular section 1040. Consequently, by the propulsive force F1, the inserting section 1002 can suitably be moved together with the spiral rotary member 1030 in the distal end direction parallel to the longitudinal axis L. Similarly, the propulsive force F2 transmitted from the rotary tubular section 1033 to the tubular main body 1031 is suitably transmitted to the inserting section 1002 through the fixing tubular section 1040. Therefore, by the propulsive force F2, the inserting section 1002 can suitably be moved together with the spiral rotary member 1030 in the proximal end direction parallel to the longitudinal axis L.

Further, in the pressed state, the pressing force acts from the luminal paries 1076 onto the spiral fin section 1037 of the rotary tubular section 1033 in the inner peripheral direction. Therefore, when the rotary tubular section 1033 in the pressed state is rotated, an anti-rotational force acts on the tubular main body 1031 in the opposite direction to the rotating direction of the rotary tubular section 1033. In the present embodiment, in the regulated state where the rotation of the tubular main body 1031 to the inserting section 1002 in the periaxial direction of the rotation axis (the periaxial direction of the longitudinal axis) is regulated, the rotary tubular section 1033 is rotated. Therefore, also when the rotary tubular section 1033 rotates in the pressed state and the anti-rotational force acts, the rotation of the tubular main body 1031 to the inserting section 1002 in the opposite direction to the rotating direction of the rotary tubular section 1033 is regulated. When the rotation of the tubular main body 1031 to the inserting section 1002 in the periaxial direction of the rotation axis is regulated, the rotary tubular section 1033 suitably rotates. Therefore, in the pressed state where the pressing force acts from the luminal paries 1076 onto the spiral fin section 1037 of the rotary tubular section 1033 in the inner peripheral direction, the rotary tubular section 1033 can suitably be rotated, and the propulsive forces F1, F2 can suitably be generated.

Furthermore, the holding tube 1039 is disposed on the outer peripheral direction side of the rotary tubular section 1033, and the holding tube 1039 can rotate to the rotary tubular section 1033 in the periaxial direction of the rotation axis. Therefore, also in a state where the rotary tubular section 1033 is rotated, the operator can hold the holding tube 1039. Further, in the tubular main body 1031, there is disposed the covering section 1069 that covers the outer peripheral direction side of the drive force transmitting section 1061 (the drive gear 1063). Therefore, in a state where the motor 1055 is driven, the drive force transmitting section 1061 (the drive gear 1063) can effectively be prevented from being touched by, e.g., an operator's hands.

Furthermore, in observation of a subject in the endoscope device 1001, an observing direction by an imaging element (not shown) is changed sometimes. In this case, by the operation in the drive operation input section 1016, the driving of the motor 1055 is stopped, and the rotating of the rotary tubular section 1033 is stopped. Furthermore, the inserting section 1002 is rotated in the periaxial direction of the longitudinal axis, thereby changing the observing direction of the imaging element. Here, when the inserting section 1002 is rotated in the periaxial direction of the longitudinal axis, the engaging protrusion 1045 of the fastening member 1042 of the fixing tubular section 1040 is engaged with the first engagement hole 1043A of the proximal end side tubular member 1041. As a result, there is obtained the non-regulated state where the rotation of the tubular main body 1031 to the inserting section 1002 in the periaxial direction of the rotation axis (the periaxial direction of the longitudinal axis) is not regulated. Also in the non-regulated state, the movement of the tubular main body 1031 to the inserting section 1002 in the direction parallel to the main body axis B (the longitudinal axis L) is regulated. The motor 1055 and the drive force transmitting section 1061 having a large weight are attached to the tubular main body 1031. Therefore, the rotation of the tubular main body 1031 to the inserting section 1002 in the periaxial direction of the longitudinal axis is not regulated, and hence the inserting section 1002 easily rotates in the periaxial direction of the longitudinal axis. As a result, also in the state where the spiral rotary member 1030 is attached to the inserting section 1002, the observing direction by the imaging element can easily be changed.

### (Modification of Second Embodiment)

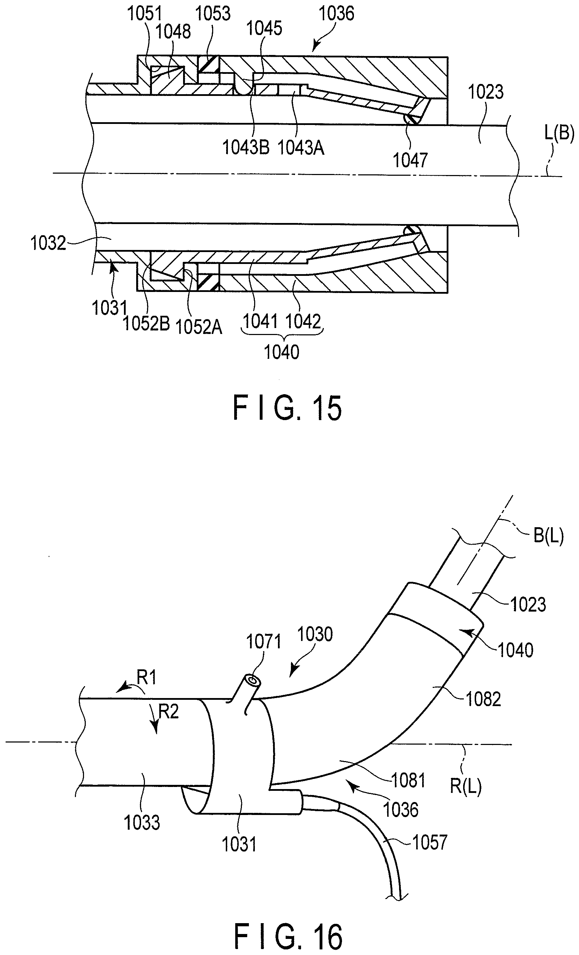

In the second embodiment, the elastic section 1053 is fixed to the tubular main body 1031, but the present invention is not restricted thereto. For example, as a first modification, the elastic section 1053 may be fixed to a fastening member 1042 as shown in FIG. 14 and FIG. 15. In the present modification, in a state where an engaging protrusion 1045 is engaged with a first engagement hole 1043A (see FIG. 14), the elastic section 1053 is not in contact with the tubular main body 1031. Therefore, there is obtained a non-regulated state where rotation of the tubular main body 1031 to an inserting section 1002 in a periaxial direction of a rotation axis is not regulated. Further, in the present modification, in a state where the engaging protrusion 1045 is engaged with a second engagement hole 1043B (see FIG. 15), the elastic section 1053 abuts on the tubular main body 1031. When the elastic section 1053 abuts on the tubular main body 1031, a friction force is generated between the elastic section 1053 and the tubular main body 1031. By the friction force, rotation of the tubular main body 1031 to a fixing tubular section 1040 in the periaxial direction of the rotation axis (a periaxial direction of a main body axis) is regulated. In consequence, there is obtained a regulated state where the rotation of the tubular main body 1031 to the inserting section 1002 in the periaxial direction of the rotation axis is regulated.

### (Third Embodiment)

Next, a third embodiment of the present invention will be described with reference to FIG. 16 to FIG. 18B. The third embodiment is obtained by modifying the configuration of the second embodiment as follows. It is to be noted that the same parts as in the second embodiment will be denoted with the same reference numerals, and description thereof will be omitted.

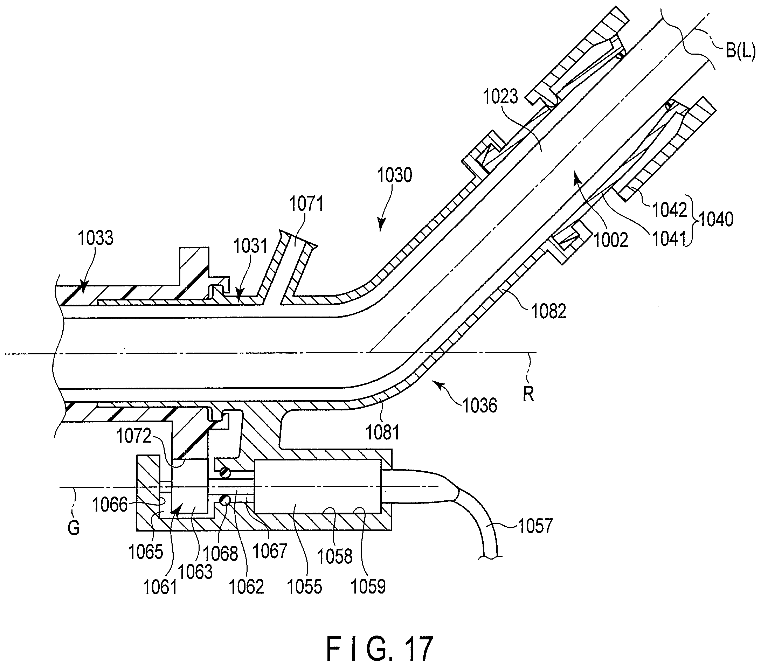

FIG. 16 and FIG. 17 are views showing a spiral rotary member 1030 according to the present embodiment. FIG. 16 and FIG. 17 show a state where the spiral rotary member 1030 is attached to an inserting section 1002. As shown in FIG. 16 and FIG. 17, also in the present embodiment, the spiral rotary member 1030 comprises a tubular main body 1031, a rotary tubular section 1033 and a fixing tubular section 1040 in the same manner as in the second embodiment. Furthermore, in a main body hollow 1032 in the state where the spiral rotary member 1030 is attached to the inserting section 1002, a main body axis B of the tubular main body 1031 is the same axis as a longitudinal axis L. Furthermore, in a tubular section hollow 1035 in the state where the spiral rotary member 1030 is attached to the inserting section 1002, a rotation axis R of the rotary tubular section 1033 is the same axis as the longitudinal axis L. Furthermore, in a distal end portion of an outer peripheral portion of the rotary tubular section 1033, a spiral fin section 1037 is disposed.

In the present embodiment, a bending section 1081 in which the main body axis B of the tubular main body 1031 is bent to the rotation axis R of the rotary tubular section 1033 in the main body hollow 1032 is disposed in the tubular main body 1031. The bending section 1081 is disposed, and hence in the present embodiment, the main body axis B of the tubular main body 1031 is not the same axis as the rotation axis R of the rotary tubular section 1033. The bending section 1081 is positioned on a proximal end direction side to a drive force transmitting section 1061. It is to be noted that also in the present embodiment, the tubular main body 1031 is made of a hard material and does not bend due to an external force.

Furthermore, in the tubular main body 1031, a tilting tubular section 1082 is disposed to be continuous with the proximal end direction side of the bending section 1081. In the tilting tubular section 1082, the main body axis B of the tubular main body 1031 tilts to the rotation axis R of the rotary tubular section 1033. That is, in the tilting tubular section 1082, the main body axis B of the tubular main body 1031 is not parallel to the rotation axis R of the rotary tubular section 1033. In the tubular main body 1031, the inserting section 1002 is bent to correspond to a shape of the tubular main body 1031.

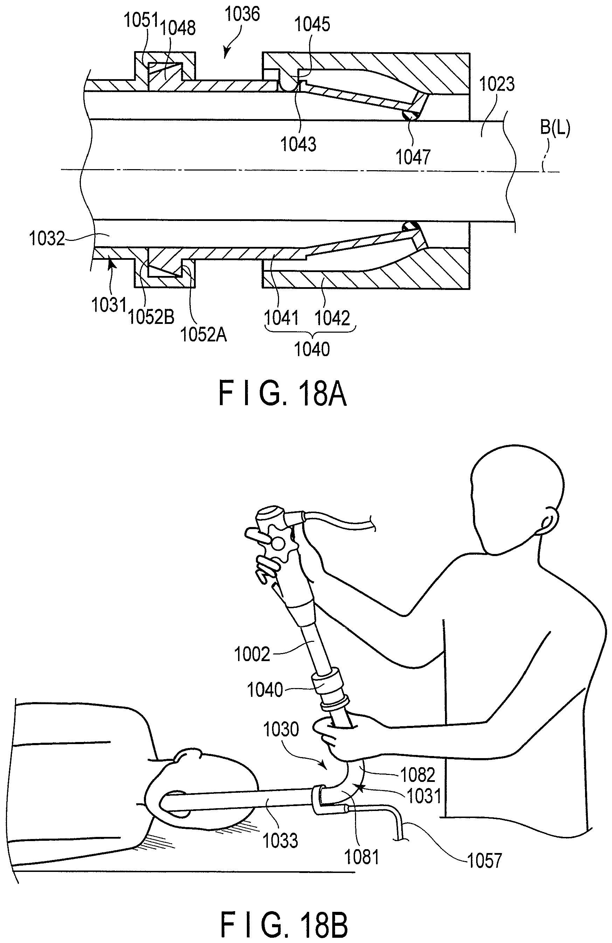

FIG. 18A is a view showing configurations of the tubular main body 1031 and the fixing tubular section 1040. As shown in FIG. 18A, in the present embodiment, only one engagement hole 1043 is disposed in a proximal end side tubular member 1041. Further, in a fastening member 1042, an engaging protrusion 1045 is disposed. When the engaging protrusion 1045 engages with the engagement hole 1043, the fastening member 1042 is fixedly attached to the proximal end side tubular member 1041. In a state where the engaging protrusion 1045 is engaged with the engagement hole 1043, a pressing force acts from the fastening member 1042 to the proximal end side tubular member 1041 in an inner peripheral direction. As a result, an elastic member 1047 contracts, and the elastic member 1047 is appressed against the proximal end side tubular member 1041 and an outer peripheral portion of the inserting section 1002. In consequence, the proximal end side tubular member 1041 is fixed to the inserting section 1002 and the fixing tubular section 1040 is fixed to the inserting section 1002.

Furthermore, also in the present embodiment, in an engaging pawl 1048 of the proximal end side tubular member 1041, abutment portions 1052A,52B that abut on the tubular main body 1031 in an engagement groove 1051 are also arranged in the same manner as in the second embodiment. Furthermore, by the abutment portions 1052A,52B of the engaging pawl 1048, movement of the tubular main body 1031 to the fixing tubular section 1040 in a direction parallel to the main body axis B (the longitudinal axis L) is regulated. Therefore, the elastic member 1047 and the abutment portions 1052A,52B function as a movement regulating section that regulates the movement of the tubular main body 1031 to the inserting section 1002 in the direction parallel to the main body axis B (the longitudinal axis L). It is to be noted that also in the present embodiment, rotation of the tubular main body 1031 in a periaxial direction of the main body axis (the periaxial direction of the longitudinal axis) is not regulated by the engaging pawl 1048.

Further, in the present embodiment, the elastic section 1053 is not disposed. Furthermore, in the state where the engaging protrusion 1045 is engaged with the engagement hole 1043, the fastening member 1042 is not in contact with the tubular main body 1031. Instead, in the present embodiment, the bending section 1081 is disposed in the tubular main body 1031. Further, in the tubular main body 1031, the inserting section 1002 is bent to correspond to the shape of the tubular main body 1031. Therefore, in the bending section 1081 of the main body hollow 1032, the longitudinal axis L of the inserting section 1002 is bent to the rotation axis R of the rotary tubular section 1033. Further, in the tilting tubular section 1082 of the tubular main body 1031, the longitudinal axis L of the inserting section 1002 tilts to the rotation axis R of the rotary tubular section 1033. As a result, rotation of the tubular main body 1031 to the inserting section 1002 in the periaxial direction of the rotation axis is regulated. That is, the bending section 1081 functions as a rotation regulating section that regulates the rotation of the tubular main body 1031 to the inserting section 1002 in the periaxial direction of the rotation axis in the state where the spiral rotary member 1030 is attached to the inserting section 1002.

However, in the present embodiment, the rotation of the tubular main body 1031 to the inserting section 1002 in the periaxial direction of the main body axis is not regulated by the bending section 1081. Therefore, also in the state where the inserting section 1002 is bent to correspond to the shape of the tubular main body 1031, the inserting section 1002 can rotate to the tubular main body 1031 in the periaxial direction of the longitudinal axis.

Next, operations and effects of an endoscope device 1001 and the spiral rotary member 1030 of the present embodiment will be described. Also in the present embodiment, in the state where the spiral rotary member 1030 is attached to the inserting section 1002, the movement of the tubular main body 1031 to the inserting section 1002 in the direction parallel to the main body axis B (the longitudinal axis L) is regulated. Therefore, propulsive force F1 transmitted from the rotary tubular section 1033 to the tubular main body 1031 is suitably transmitted to the inserting section 1002 through the fixing tubular section 1040. Therefore, by the propulsive force F1, the inserting section 1002 can suitably be moved together with the spiral rotary member 1030 in a distal end direction parallel to the longitudinal axis L. Similarly, propulsive force F2 transmitted from the rotary tubular section 1033 to the tubular main body 1031 is suitably transmitted to the inserting section 1002 through the fixing tubular section 1040. Therefore, by the propulsive force F2, the inserting section 1002 can suitably be moved together with the spiral rotary member 1030 in a proximal end direction parallel to the longitudinal axis L.

Further, in the present embodiment, the rotation of the tubular main body 1031 to the inserting section 1002 in the periaxial direction of the rotation axis is regulated by the bending section 1081. Therefore, also when the rotary tubular section 1033 rotates in a pressed state and an anti-rotational force acts, rotation of the tubular main body 1031 to the inserting section 1002 in an opposite direction to a rotating direction of the rotary tubular section 1033 is regulated. When the rotation of the tubular main body 1031 to the inserting section 1002 in the periaxial direction of the rotation axis is regulated, the rotary tubular section 1033 suitably rotates. Therefore, in the pressed state where a pressing force acts from a luminal paries 1076 onto the spiral fin section 1037 of the rotary tubular section 1033 in the inner peripheral direction, the rotary tubular section 1033 can suitably be rotated, and the propulsive forces F1, F2 can suitably be generated.

Furthermore, the rotation of the tubular main body 1031 to the inserting section 1002 in the periaxial direction of the main body axis is not regulated by the bending section 1081. Therefore, also in a state where the inserting section 1002 is bent to correspond to the shape of the tubular main body 1031, the inserting section 1002 can rotate to the tubular main body 1031 in the periaxial direction of the longitudinal axis. The motor 1055 and the drive force transmitting section 1061 having a large weight are attached to the tubular main body 1031. In consequence, the rotation of the tubular main body 1031 to the inserting section 1002 in the periaxial direction of the longitudinal axis is not regulated, and hence the inserting section 1002 easily rotates in the periaxial direction of the longitudinal axis. As a result, also when the spiral rotary member 1030 is attached to the inserting section 1002, a direction in which an imaging element observes can easily be changed.

Further, as shown in FIG. 18B, in a state where the inserting section 1002 and the spiral rotary member 1030 are inserted from, e.g., a mouth into a lumen, the inserting section 1002 and the spiral rotary member 1030 are bent more than once outside of a body (on a proximal end side to the mouth). In the present embodiment, the bending section 1081 is disposed on the proximal end direction side to the drive force transmitting section 1061, and the inserting section 1002 and the spiral rotary member 1030 are bent by the bending section 1081. That is, by the bending section 1081, the inserting section 1002 and the spiral rotary member 1030 are bent outside of the body. A rotary drive force to be transmitted from the drive force transmitting section 1061 may be transmitted to a distal end direction side from the drive force transmitting section 1061. In consequence, even when the inserting section 1002 and the spiral rotary member 1030 are bent by the bending section 1081 positioned on the proximal end direction side of the drive force transmitting section 1061, transmissibility of the rotary drive force to be transmitted up to the distal end portion of the rotary tubular section 1033 is not influenced. That is, the transmissibility of the rotary drive force is not deteriorated when the inserting section 1002 and the spiral rotary member 1030 are bent by the bending section 1081. Therefore, when the bending section 1081 is disposed on the proximal end direction side of the drive force transmitting section 1061, the transmissibility of the rotary drive force is assured, so that the rotary drive force can suitably be transmitted up to the distal end portion of the rotary tubular section 1033.

### (Other Modifications)

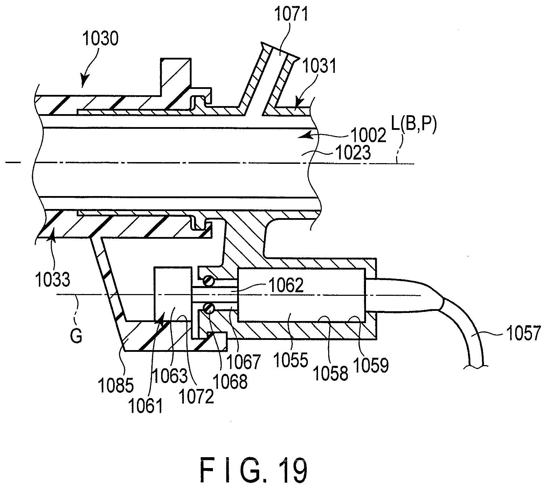

In the abovementioned embodiments, the outer peripheral direction side of the drive force transmitting section 1061 (the drive gear 1063) is covered with the covering section 1069 of the tubular main body 1031, but the present invention is not restricted thereto. For example, as a second modification, as shown in FIG. 19, in a rotary tubular section 1033, a covering section 1085 that covers an outer peripheral direction side of a drive force transmitting section 1061 (a drive gear 1063) may be disposed. In the present modification, the gear hollow portion 1065 is not disposed, and the drive force transmitting section 1061 (the drive gear 1063) is positioned outside of a tubular main body 1031. Also in the present modification, a seal ring 1068 is disposed in a communication passage 1067, and the seal ring 1068 maintains liquid-tightness between a shaft member 1062 and the tubular main body 1031. As a result, liquid is prevented from flowing from the outside of the tubular main body 1031 into a member hollow portion 1058.

The covering section 1085 is disposed, hence also in the present modification, the drive force transmitting section 1061 (the drive gear 1063) can effectively be prevented from being touched by an operator's hands or the like in a state where the motor 1055 is driven. Furthermore, after use of a spiral rotary member 1030, the rotary tubular section 1033 is discarded, and the tubular main body 1031 and the drive force transmitting section 1061 are cleaned and reused. In the present embodiment, the gear hollow portion 1065 is not disposed in the tubular main body 1031, and the drive force transmitting section 1061 (the drive gear 1063) is positioned outside of the tubular main body 1031. Therefore, cleanability of the tubular main body 1031 and the drive force transmitting section 1061 improves.

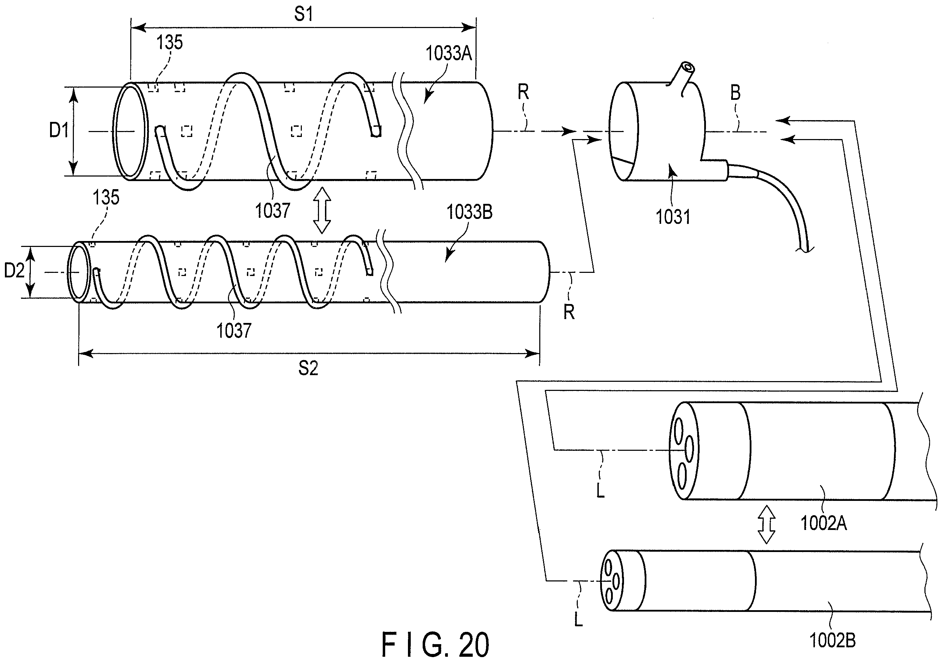

Furthermore, as a third modification, as shown in FIG. 20, a first rotary tubular section 1033A and a second rotary tubular section 1033B having a specification different from that of the first rotary tubular section 1033A can selectively be attached to a tubular main body 1031. The first rotary tubular section 1033A has a first inner diameter D1, and has a first axially parallel dimension S1 in a direction parallel to a rotation axis R. The second rotary tubular section 1033B has a second inner diameter D2 smaller than the first inner diameter D1, and has a second axially parallel dimension S2 larger than the first axially parallel dimension S1 in the direction parallel to the rotation axis R. Furthermore, the first rotary tubular section 1033A is different from the second rotary tubular section 1033B in a pitch of a spiral fin section 1037. The first rotary tubular section 1033A is used in observation of, e.g., a large intestine. The second rotary tubular section 1033B is used in observation of, e.g., a small intestine. As described above, the rotary tubular sections (1033A, 1033B) having different specifications can selectively be attached to the tubular main body 1031.

Further, in the present modification, the tubular main body 1031 can selectively be attached to a first inserting section 1002A and a second inserting section 1002B having a specification different from that of the first inserting section 1002A. The first inserting section 1002A is used in the observation of, e.g., the large intestine. The second inserting section 1002B is used in the observation of, e.g., the small intestine. That is, the tubular main body 1031 can selectively be attached to the inserting sections (1002A, 1002B) having different specifications.

In the present modification, the rotary tubular sections (1033A, 1033B) having different specifications can selectively be attached to one tubular main body 1031. Furthermore, the one tubular main body 1031 can selectively be attached to the inserting sections (1002A, 1002B) having different specifications. That is, the tubular main body 1031 does not have to be replaced in accordance with a change in the specifications of the rotary tubular sections (1033A, 1033B) and the inserting sections (1002A, 1002B). That is, even when the specifications of the rotary tubular sections (1033A, 1033B) and the inserting sections (1002A, 1002B) change in accordance with an observation region and a type of patient, the tubular main body 1031 does not have to be replaced. Therefore, the spiral rotary member 1030 can be used in various observation regions and various patients without raising costs.

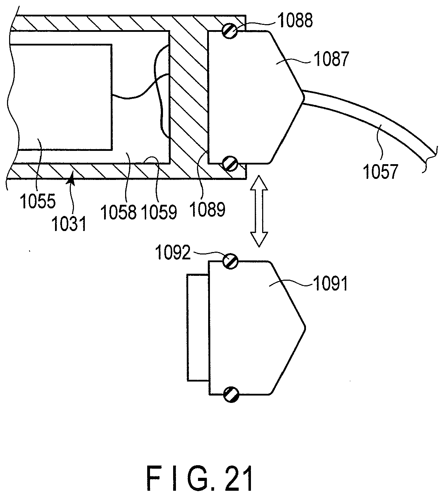

Furthermore, as a fourth modification, as shown in FIG. 21, a motor cable (a cable) 1057 may be detachably attached to a tubular main body 1031. In the present modification, a connection plug 1087 is disposed at one end of the motor cable 1057. A seal ring 1088 is attached to the connection plug 1087. Furthermore, in the tubular main body 1031, a socket section 1089 which is a cable connecting section is disposed. The connection plug 1087 is detachably attached to the socket section 1089. That is, the motor cable 1057 is detachably attached to the socket section 1089.

When the motor cable 1057 is connected to the socket section 1089, power can be supplied to a motor 1055. Further, in a state where the motor cable 1057 is connected to the socket section 1089, the seal ring 1088 maintains liquid-tightness between the tubular main body 1031 and the connection plug 1087. Therefore, liquid is prevented from flowing from the outside of the tubular main body 1031 into a member hollow portion 1058.

Furthermore, a liquid-proof cap 1091 as a liquid-proof member is detachably attached to the socket section 1089 as the cable connecting section. A seal ring 1092 is attached to the liquid-proof cap 1091. In a state where the liquid-proof cap 1091 is attached to the socket section 1089, the seal ring 1092 maintains liquid-tightness between the tubular main body 1031 and the liquid-proof cap 1091. Therefore, liquid is prevented from flowing from the outside of the tubular main body 1031 into the member hollow portion 1058.

The motor 1055 as a drive member is attached to the tubular main body 1031 of the spiral rotary member 1030, hence autoclave sterilization cannot easily be performed. Therefore, the tubular main body 1031 is cleaned and disinfected by an endoscope cleaner. In the present modification, when the tubular main body 1031 is cleaned and disinfected, the connection plug 1087 (the motor cable 1057) is removed from the socket section 1089. Then, the liquid-proof cap 1091 is attached to the socket section 1089. In this state, the cleaning and disinfection by the endoscope cleaner are performed.

During the cleaning and disinfection of the tubular main body 1031, the motor cable 1057 is removed from the socket section 1089 of the tubular main body 1031, hence the cleaning and disinfection by the endoscope cleaner can easily be performed. Further, in the state where the liquid-proof cap 1091 is attached to the socket section 1089, the liquid-tightness is kept between the liquid-proof cap 1091 and the tubular main body 1031. As a result, during sterilization of the tubular main body 1031, the liquid is prevented from flowing from the outside of the tubular main body 1031 into the member hollow portion 1058.

As described above, in the embodiments and modifications of the present invention, the spiral rotary member 1030 comprises the tubular main body 1031 having the main body axis B which is the same axis as the longitudinal axis L in the main body hollow 1032 attached to the inserting section 1002, and the rotary tubular section 1033 having the rotation axis R which is the same axis as the longitudinal axis L in the tubular section hollow 1035 attached to the inserting section 1002. Furthermore, the rotary tubular section 1033 is attached to the distal end direction side of the tubular main body 1031 so as to be rotatable to the tubular main body 1031 in the periaxial direction of the rotation axis. Further, in the outer peripheral portion of the rotary tubular section 1033, the spiral fin section 1037 protruding toward the outer peripheral direction spirally extends along the rotation axis R. Furthermore, the drive member (1055) that is driven when the power is supplied thereto is attached to the tubular main body 1031. The rotary drive force produced by driving of the drive member (1055) is transmitted to the rotary tubular section 1033 by the drive force transmitting section 1061. Furthermore, the spiral rotary member 1030 comprises the movement regulating section (1047, 1052A, 1052B) that regulates the movement of the tubular main body 1031 to the inserting section 1002 in the direction parallel to the main body axis B in the state where the spiral rotary member is attached to the inserting section 1002, and the rotation regulating section (1047, 1053; 1081) that regulates the rotation of the tubular main body 1031 to the inserting section 1002 in the periaxial direction of the rotation axis in the state where the spiral rotary member is attached to the inserting section 1002.

The present invention is not restricted to the foregoing embodiments as they are, and constituent elements can be modified and embodied without departing from the gist in embodying stages. Additionally, various inventions can be formed by appropriately combining constituent elements disclosed in the foregoing embodiments.

### [Additional Notes]

### [Additional Note 1]

An auxiliary insertion device that allows an inserting section extending along a longitudinal axis to be inserted thereinto and is attached to the inserting section, the auxiliary insertion device comprising:
a tubular main body including a main body hollow formed on an inner peripheral direction side, and having a main body axis which is the same axis as the longitudinal axis in the main body hollow in a state where the auxiliary insertion device is attached to the inserting section;
a rotary tubular section including a tubular section hollow formed on the inner peripheral direction side, having a rotation axis which is the same axis as the longitudinal axis in the tubular section hollow in the state where the auxiliary insertion device is attached to the inserting section, and attached to a distal end direction side of the tubular main body in a state where the rotary tubular section is rotatable to the tubular main body in a periaxial direction of the rotation axis;
a spiral fin section disposed on an outer peripheral portion of the rotary tubular section in a state where the spiral fin section is projected toward an outer peripheral direction, and spirally extending along the rotation axis;
a drive member that is attached to the tubular main body and driven when power is supplied thereto;
a drive force transmitting section that transmits a rotary drive force produced by driving of the drive member to the rotary tubular section in the state where the auxiliary insertion device is attached to the inserting section, thereby rotating the rotary tubular section to the inserting section in the periaxial direction of the rotation axis;
a movement regulating section that regulates movement of the tubular main body to the inserting section in a direction parallel to the main body axis in the state where the auxiliary insertion device is attached to the inserting section; and
a rotation regulating section that regulates rotation of the tubular main body to the inserting section in the periaxial direction of the rotation axis in the state where the auxiliary insertion device is attached to the inserting section.

### [Additional Note 2]

The auxiliary insertion device of additional note 1, further comprising:
a regulation switching section that switches the rotation regulating section to a non-regulated state where the rotation of the tubular main body to the inserting section in the periaxial direction of the rotation axis is not regulated and a regulated state where the rotation of the tubular main body to the inserting section in the periaxial direction of the rotation axis is regulated, in the state where the auxiliary insertion device is attached to the inserting section.

### [Additional Note 3]

The auxiliary insertion device of additional note 2, further comprising:
a fixing tubular section attached to the tubular main body, and fixed to the inserting section in the state where the auxiliary insertion device is attached to the inserting section,
wherein the regulation switching section switches the tubular main body to a state where the tubular main body is rotatable to the fixing tubular section in the periaxial direction of the rotation axis in the non-regulated state, and switches the tubular main body to a state where the tubular main body is not rotatable to the fixing tubular section in the periaxial direction of the rotation axis in the regulated state.

### [Additional Note 4]

The auxiliary insertion device of additional note 3,
wherein the movement regulating section comprises an abutment portion that is abuttable on the tubular main body and the fixing tubular section and regulates such that the tubular main body moves in the direction parallel to the main body axis.

### [Additional Note 5]

The auxiliary insertion device of additional note 3,
wherein the rotation regulating section comprises an elastic section that is interposed between the tubular main body and the fixing tubular section and generates a friction force, thereby regulating the rotation of the tubular main body in the periaxial direction of the rotation axis in the regulated state.

### [Additional Note 6]

The auxiliary insertion device of additional note 1,
wherein the rotation regulating section comprises a bending section that is disposed on a proximal end direction side to the drive force transmitting section and bends the main body axis of the tubular main body to the rotation axis in the main body hollow, and
the tubular main body comprises a tilting tubular section that is continuous with the proximal end direction side of the bending section and in which the main body axis tilts to the rotation axis.

### [Additional Note 7]

The auxiliary insertion device of additional note 6, further comprising:
a fixing tubular section attached to the tubular main body and fixed to the inserting section in the state where the auxiliary insertion device is attached to the inserting section,
wherein the movement regulating section comprises an abutment portion that is abuttable on the tubular main body and the fixing tubular section and regulates such that the tubular main body moves in the direction parallel to the main body axis.

### [Additional Note 8]

The auxiliary insertion device of additional note 1,
wherein the rotary tubular section comprises a drive force receiving section that receives the rotary drive force from the drive force transmitting section,
the drive force transmitting section comprises a drive gear that rotates in a periaxial direction of a gear axis, when the drive member is driven, and
the drive force receiving section comprises a rotation side gear section that meshes with the drive gear.

### [Additional Note 9]

The auxiliary insertion device of additional note 1,
wherein the rotary tubular section comprises a drive force receiving section that receives the rotary drive force from the drive force transmitting section,
the tubular main body comprises a member hollow defining section that defines a member hollow portion in which the drive member is received,
the drive force transmitting section is positioned outside of the tubular main body, and
the rotary tubular section comprises a covering section that covers the outer peripheral direction side of the drive force transmitting section.

### [Additional Note 10]

The auxiliary insertion device of additional note 1,
wherein the rotary tubular section comprises a first rotary tubular section and a second rotary tubular section having a specification different from that of the first rotary tubular section, and
the tubular main body is selectively attachable to the first rotary tubular section and the second rotary tubular section.

### [Additional Note 11]

The auxiliary insertion device of additional note 1, further comprising:
a cable that supplies power to the drive member,
wherein the tubular main body comprises a member hollow defining section that defines a member hollow portion in which the drive member is received, and a cable connecting section detachably connected to the cable.

### [Additional Note 12]

The auxiliary insertion device of additional note 11, further comprising:
a liquid-proof member that is detachably attached to the cable connecting section of the tubular main body and prevents liquid from flowing from an outside of the tubular main body into the member hollow portion in a state where the liquid-proof member is attached to the cable connecting section.

### [Additional Note 13]

An insertion device comprising:
the auxiliary insertion device of additional note 1; and
the inserting section to which the auxiliary insertion device is attached.

### [Additional Note 14]

A rotary tubular section that is attached as a rotation target to a drive source unit disposed in an auxiliary insertion device that allows an inserting section extending along a longitudinal axis to be inserted thereinto and is attached to the inserting section, the drive source unit comprising a tubular main body including a main body hollow formed on an inner peripheral direction side and having a main body axis which is the same axis as the longitudinal axis in the main body hollow in a state where the auxiliary insertion device is attached to the inserting section, a drive member that is attached to the tubular main body and driven when power is supplied thereto, a drive force transmitting section that transmits a rotary drive force produced by driving of the drive member to a rotation target, thereby rotating the rotation target to the inserting section in a periaxial direction of a rotation axis in the state where the auxiliary insertion device is attached to the inserting section, a movement regulating section that regulates movement of the tubular main body to the inserting section in a direction parallel to the main body axis in the state where the auxiliary insertion device is attached to the inserting section, and a rotation regulating section that regulates rotation of the tubular main body to the inserting section in the periaxial direction of the rotation axis in the state where the auxiliary insertion device is attached to the inserting section,
wherein on the inner peripheral direction side of the rotary tubular section, a tubular section hollow is formed,
the rotary tubular section having the rotation axis which is the same axis as the longitudinal axis in the tubular section hollow in the state where the auxiliary insertion device is attached to the inserting section, the rotary tubular section being attached to a distal end direction side of the tubular main body in a state where the rotary tubular section is rotatable to the tubular main body in the periaxial direction of the rotation axis,
the rotary tubular section comprising a spiral fin section spirally extending on an outer peripheral portion of the rotary tubular section along the rotation axis in a state where the spiral fin section is projected toward an outer peripheral direction.

### [Additional Note 15]

A drive source unit disposed in an auxiliary insertion device that allows an inserting section extending along a longitudinal axis to be inserted thereinto and is attached to the inserting section, and attached to a rotary tubular section including a tubular section hollow formed on an inner peripheral direction side, having a rotation axis which is the same axis as the longitudinal axis in the tubular section hollow in a state where the auxiliary insertion device is attached to the inserting section and comprising a spiral fin section disposed on an outer peripheral portion in a state where the spiral fin section is projected toward an outer peripheral direction and spirally extending along the rotation axis, the drive source unit comprising:
a tubular main body including a main body hollow formed on the inner peripheral direction side and having a main body axis which is the same axis as the longitudinal axis in the main body hollow in the state where the auxiliary insertion device is attached to the inserting section, the rotary tubular section being attached to a distal end direction side in a state where the rotary tubular section is rotatable in a periaxial direction of the rotation axis;
a drive member that is attached to the tubular main body and driven when power is supplied thereto;
a drive force transmitting section that transmits a rotary drive force produced by driving of the drive member to the rotary tubular section, thereby rotating the rotary tubular section to the inserting section in the periaxial direction of the rotation axis, in the state where the auxiliary insertion device is attached to the inserting section;
a movement regulating section that regulates movement of the tubular main body to the inserting section in a direction parallel to the main body axis in the state where the auxiliary insertion device is attached to the inserting section; and
a rotation regulating section that regulates rotation of the tubular main body to the inserting section in the periaxial direction of the rotation axis in the state where the auxiliary insertion device is attached to the inserting section.

## Claims

1. An auxiliary insertion and removal device (130) that is configured to be inserted into or removed from a lumen in a state where an inserting section (30) of an endoscope (20) having a longitudinal axis is inserted thereinto and that assists in the insertion and the removal of the inserting section (30) into and from the lumen, the auxiliary insertion and removal device (130) comprising:
a tubular main body section (131) that allows the inserting section (30) to be inserted thereinto and is configured to rotate around the longitudinal axis;
a fin section (133) that is disposed on an outer peripheral surface of the main body section (131) and spirally arranged around the longitudinal axis; and
X-ray cutoff sections (135) that are arranged in at least a part of the auxiliary insertion and removal device (130) so that a clear region to be clearly imaged by X-rays and an unclear region to be unclearly imaged by X-rays are distinguishable under X-ray observation, wherein the X-ray cutoff sections (135) are arranged on a straight line along the longitudinal axis direction of the auxiliary insertion and removal device (130),
the respective X-ray cut-off sections (135) being further arranged in the main body section (131) between spiral turns of the fin section (133) in the longitudinal axis direction of the auxiliary insertion and removal device (130), and
wherein the X-ray cutoff sections (135) are further arranged in a periaxial direction of the longitudinal axis of the auxiliary insertion and removal device (130), one of the X-ray cutoff sections (135) being disposed about 90 degree away from the other of the X-ray cutoff sections (135) in the periaxial direction.

2. The auxiliary insertion and removal device (130) according to claim 1,
wherein the X-ray cutoff sections (135) are disposed in at least one of a proximal end portion of the main body section (131), a proximal end portion side attaching portion of the main body section (131) to which a proximal end portion of the fin section (133) is attached, and the proximal end portion of the fin section (133).

3. The auxiliary insertion and removal device (130) according to claim 1,
wherein the X-ray cutoff sections (135) are disposed in at least one of a distal end portion of the main body section (131), a distal end portion side attaching portion of the main body section (131) to which a distal end portion of the fin section (133) is attached, and the distal end portion of the fin section (133).

4. The auxiliary insertion and removal device (130) according to claim 1,
wherein the inserting section (30) has a rotary member (110) that rotates around the longitudinal axis by a transmitted drive force, and
the main body section (131) is detachably attached to an outer peripheral surface of the rotary member (110) so that the main body section (131) rotates around the longitudinal axis in accordance with the rotation of the rotary member (110).

5. The auxiliary insertion and removal device (130) according to claim 1,
wherein the main body section (1031) has a main body hollow formed (1032) on an inner peripheral direction side, and a main body axis which is the same axis as a longitudinal axis in the main body hollow (1032) in a state where the main body section (1030) is attached to the inserting section (1002),
the auxiliary insertion and removal device (130) further comprising:
a rotary tubular section (1033) including a tubular section hollow (1035) formed on the inner peripheral direction side, and having a rotation axis which is the same axis as the longitudinal axis in the tubular section hollow (1035) in the state where the main body section (1031) is attached to the inserting section (1002), the rotary tubular section (1033) being attached to a distal end direction side of the main body section (1031) in a state where the rotary tubular section (1033) is rotatable to the main body section (1031) in a periaxial direction of the rotation axis;
a drive member (1055) that is attached to the main body section (1031) and driven when power is supplied thereto;
a drive force transmitting section (1061) that transmits a rotary drive force produced by driving of the drive member (1055) to the rotary tubular section (1033) in the state where the main body section (1031) is attached to the inserting section (1002), thereby rotating the rotary tubular section (1033) to the inserting section (1002) in the periaxial direction of the rotation axis;
a movement regulating section (1047, 1052A, 1052B) that regulates movement of the main body section (1031) to the inserting section (1002) in a direction parallel to the main body axis in the state where the main body section (1031) is attached to the inserting section (1002); and
a rotation regulating section (1047, 1053, 1081) that regulates rotation of the main body section (1031) to the inserting section (1002) in the periaxial direction of the rotation axis in the state where the main body section (1031) is attached to the inserting section (1002).

## Patentansprüche

1. Hilfseinführ- und Entfernungsgerät (130), das dazu ausgebildet ist, in ein Lumen in einem Zustand eingeführt oder aus diesem entfernt zu werden, in welchem ein Einführabschnitt (30) eines eine Longitudinalachse aufweisendes Endoskops (20) darin eingeführt wird und das bei dem Einführen und dem Entfernen des Einführabschnitts (30) in oder aus dem Lumen unterstützt, wobei das Hilfseinführ- und Entfernungsgerät (130) umfasst:
einen rohrförmigen Hauptkörperabschnitt (131), der es dem Einführungsabschnitt (30) erlaubt, darin eingeführt zu werden, und der zum Rotieren um die Longitudinalachse ausgebildet ist;
einen Lamellenabschnitt (133), der an einer äußeren Umfangsfläche des Hauptkörperabschnitts (131) angeordnet ist und spiralförmig um die Longitudinalachse angeordnet ist; und
Röntgenstrahl-Ausschnittabschnitte (135), die in wenigstens einem Teil des Hilfseinführ- und Entfernungsgerät (130) angeordnet sind, sodass eine durch Röntgenstrahlen deutlich abzubildende deutliche Region und eine durch Röntgenstrahlen undeutlich abzubildende undeutliche Region unter Röntgenstrahlbetrachtung unterscheidbar sind,
wobei die Röntgenstrahl-Ausschnittabschnitte (135) auf einer geraden Linie entlang der Longitudinalachsenrichtung des Hilfseinführ- und Entfernungsgeräts (130) angeordnet sind,
die jeweiligen Röntgenstrahl-Ausschnittabschnitte (135) weiter in dem Hauptkörperabschnitt (131) zwischen Wendeln des Lamellenabschnitts (133) in der Longitudinalachsenrichtung des Hilfseinführ- und Entfernungsgeräts (130) angeordnet sind, und
wobei die Röntgenstrahl-Ausschnitabschnitte (135) weiter in einer Periaxialrichtung der Longitudinalachse des Hilfseinführ- und Entfernungsgeräts (130) angeordnet sind,
wobei eine der Röntgenstrahl-Ausschnittabschnitte (135) um 90 Grad versetzt von den anderen der Röntgenstrahl-Ausschnittabschnitte (135) in der Periaxialrichtung angeordnet ist.

2. Hilfseinführ- und Entfernungsgerät (130) nach Anspruch 1,
wobei die Röntgenstrahl-Ausschnittabschnitte (135) in wenigstens einem von einem proximalen Endbereich des Hauptkörperabschnitts (131), einem proximalen Endbereichsseitenanschlussbereich des Hauptkörperabschnitts (131), an dem ein proximaler Endbereich des Lamellenabschnitts (133) angebracht ist, und dem proximalen Endbereich des Lamellenabschnitts (133) angeordnet sind.

3. Hilfseinführ- und Entfernungsgerät (130) nach Anspruch 1,
wobei die Röntgenstrahl-Ausschnittabschnitte (135) in wenigstens einem von einem distalen Endbereich des Hauptkörperabschnitts (131), einem distalen Endbereichsseitenanschlussbereich des Hauptkörperabschnitts (131), an dem ein distaler Endbereich des Lamellenabschnitts (133) angebracht ist, und dem distalen Endbereich des Lamellenabschnitts (133) angeordnet ist.

4. Hilfseinführ- und Entfernungsgerät (130) nach Anspruch 1,
wobei der Einführabschnitt (30) eine Dreheinheit (110) aufweist, die sich um die Longitudinalachse durch eine übertragene Antriebskraft dreht, und
der Hauptkörperabschnitt (131) abnehmbar an einer äußeren Umfangsfläche der Dreheinheit (110) angebracht ist, sodass der Hauptkörperabschnitt (131) um die Longitudinalachse entsprechend der Rotation der Dreheinheit (110) dreht.

5. Hilfseinführ- und Entfernungsgerät (130) nach Anspruch 1,
wobei der Hauptkörperabschnitt (131) einen an einer inneren Umfangsrichtungsseite hohl geformten Hauptkörper (1032), und eine Hauptkörperachse aufweist, die dieselbe Achse wie eine Longitudinalachse des hohlen Hauptkörpers (1032) in einem Zustand ist, in dem der Hauptkörperabschnitt (1030) an dem Einführabschnitt (1002) angebracht ist,
das Hilfseinführ- und Entfernungsgerät (130) weiter umfasst:
einen drehbaren Rohrabschnitt (1033), der einen auf der inneren Umfangsrichtungsseite geformten hohlen Rohrabschnitt (1035) umfasst, und eine Rotationsachse aufweist, die dieselbe Achse wie die Longitudinalachse in dem hohlen Rohrabschnitt (1035) in dem Zustand ist, in welchem der Hauptkörperabschnitt (1031) an dem Einführabschnitt (1002) angebracht ist, wobei der drehbare Rohrabschnitt (1033) an einer distalen Endrichtungsseite des Hauptkörperabschnitts (1031) in einem Zustand angebracht ist, in welchem der drehbare Rohrabschnitt (1033) zu dem Hauptkörperabschnitt (1031) in eine Periaxialrichtung der Rotationsachse drehbar ist;
eine Antriebseinheit (1055), die an dem Hauptkörperabschnitt (1031) angebracht ist und angetrieben wird, wenn diese mit Energie versorgt wird;
eine Antriebskraftübertragungseinheit (1061), die eine Drehantriebskraft überträgt, in dem die Antriebseinheit (1055) zu dem drehbaren Rohrabschnitt (1033) in dem Zustand betrieben wird, in welchem der Hauptkörperabschnitt (1031) an dem Einführabschnitt (1002) angebracht ist, um somit den drehbaren Rohrabschnitt (1033) zu dem Einführabschnitt (1002) in die Periaxialrichtung der Rotationsachse zu drehen;
eine Bewegungsregulationseinheit (1047, 1052A, 1052B), die eine Bewegung des Hauptkörperabschnitts (1031) zu dem Einführabschnitt (1002) in eine Richtung parallel zu der Hauptkörperachse in dem Zustand reguliert, in welchem der Hauptkörperabschnitt (1031) an dem Einführabschnitt (1002) angebracht ist; und
eine Drehregulationseinheit (1047, 1053, 1081), die eine Rotation des Hauptkörperabschnitts (1031) zu dem Einführabschnitt (1002) in die Periaxialrichtung der Rotationsachse in dem Zustand reguliert, in welchem der Hauptkörperabschnitt (1031) an dem Einführabschnitt (1002) angebracht ist.

## Revendications

1. Dispositif d'introduction et de retrait auxiliaire (130) qui est configuré pour être introduit dans une lumière ou retiré de celle-ci dans un état où une section d'introduction (30) d'un endoscope (20) ayant un axe longitudinal est introduite dans celle-ci et qui aide à l'introduction de la section d'introduction (30) dans la lumière et à son retrait de celle-ci, le dispositif d'introduction et de retrait auxiliaire (130) comprenant :
une section de corps principal tubulaire (131) qui permet à la section d'introduction (30) d'être introduite dans celle-ci et est configurée pour tourner autour de l'axe longitudinal ;
une section d'ailette (133) qui est disposée sur une surface périphérique externe de la section de corps principal (131) et arrangée en spirale autour de l'axe longitudinal ; et
des sections de coupure de rayons X (135) qui sont arrangées dans au moins une partie du dispositif d'introduction et de retrait auxiliaire (130) de telle sorte qu'une région distincte à imager de façon distincte par rayons X et une région indistincte à imager de façon indistincte par rayons X sont distinguables par observation aux rayons X,
les sections de coupure de rayons X (135) étant arrangées sur une ligne droite le long de la direction d'axe longitudinal du dispositif d'introduction et de retrait auxiliaire (130),
les sections de coupure de rayons X respectives (135) étant en outre arrangées dans la section de corps principal (131) entre des tours de spirale de la section d'ailette (133) dans la direction d'axe longitudinal du dispositif d'introduction et de retrait auxiliaire (130), et
les sections de coupure de rayons X (135) étant en outre arrangées dans une direction péri-axiale de l'axe longitudinal du dispositif d'introduction et de retrait auxiliaire (130),
une des sections de coupure de rayons X (135) étant disposée à environ 90 degrés de l'autre des sections de coupure de rayons X (135) dans la direction péri-axiale.

2. Dispositif d'introduction et de retrait auxiliaire (130) selon la revendication 1,
dans lequel les sections de coupure de rayons X (135) sont disposées dans au moins l'une d'une partie d'extrémité proximale de la section de corps principal (131), d'une partie d'attache côté partie d'extrémité proximale de la section de corps principal (131) à laquelle une partie d'extrémité proximale de la section d'ailette (133) est attachée, et de la partie d'extrémité proximale de la section d'ailette (133).

3. Dispositif d'introduction et de retrait auxiliaire (130) selon la revendication 1,
dans lequel les sections de coupure de rayons X (135) sont disposées dans au moins l'une d'une partie d'extrémité distale de la section de corps principal (131), d'une partie d'attache côté partie extrémité distale de la section de corps principal (131) à laquelle une partie d'extrémité distale de la section d'ailette (133) est attachée, et de la partie d'extrémité distale de la section d'ailette (133).

4. Dispositif d'introduction et de retrait auxiliaire (130) selon la revendication 1,
dans lequel la section d'introduction (30) a un élément rotatif (110) qui tourne autour de l'axe longitudinal par une force d'entraînement transmise ; et
la section de corps principal (131) est attachée de manière détachable à une surface périphérique externe de l'élément rotatif (110) de telle sorte que la section de corps principal (131) tourne autour de l'axe longitudinal conformément à la rotation de l'élément rotatif (110).

5. Dispositif d'introduction et de retrait auxiliaire (130) selon la revendication 1,
dans lequel la section de corps principal (1031) a un creux de corps principal formé (1032) sur un côté de direction périphérique interne, et un axe de corps principal qui est le même axe qu'un axe longitudinal dans le creux de corps principal (1032) dans un état où la section de corps principal (1030) est attachée à la section d'introduction (1002),
le dispositif d'introduction et de retrait auxiliaire (130) comprenant en outre :
une section tubulaire rotative (1033) comprenant un creux de section tubulaire (1035) formé sur le côté de direction périphérique interne, et ayant un axe de rotation qui est le même axe que l'axe longitudinal dans le creux de section tubulaire (1035) dans l'état où la section de corps principal (1031) est attachée à la section d'introduction (1002), la section tubulaire rotative (1033) étant attachée à un côté de direction d'extrémité distale de la section de corps principal (1031) dans un état où la section tubulaire rotative (1033) est apte à tourner par rapport à la section de corps principal (1031) dans une direction péri-axiale de l'axe de rotation ;
un élément d'entraînement (1055) qui est attaché à la section de corps principal (1031) et entraîné lorsqu'une puissance est fournie à celui-ci ;
une section de transmission de force d'entraînement (1061) qui transmet une force d'entraînement en rotation produite par l'entraînement de l'élément d'entraînement (1055) à la section tubulaire rotative (1033) dans l'état où la section de corps principal (1031) est attachée à la section d'introduction (1002), permettant ainsi de faire tourner la section tubulaire rotative (1033) par rapport à la section d'introduction (1002) dans la direction péri-axiale de l'axe de rotation ;
une section de régulation de mouvement (1047, 1052A, 1052B) qui régule un mouvement de la section de corps principal (1031) par rapport à la section d'introduction (1002) dans une direction parallèle à l'axe de corps principal dans l'état où la section de corps principal (1031) est attachée à la section d'introduction (1002) ; et
une section de régulation de rotation (1047, 1053, 1081) qui régule la rotation de la section de corps principal (1031) par rapport à la section d'introduction (1002) dans la direction péri-axiale de l'axe de rotation dans l'état où la section de corps principal (1031) est attachée à la section d'introduction (1002).
